# EUROPEAN PATENT APPLICATION

(11) **EP 2 404 544 A1**
(43) Date of publication of application: **11.01.2012**
(21) Application number: 11173307.7
(22) Date of filing: 08.07.2011
(51) Int. Cl.: A61B 1/07, A61B 1/06, A61B 1/05

(54) **Endoscope apparatus**

(30) Priority: 09.07.2010 JP 2010157357
(71) Applicant: FUJIFILM Corporation, Tokyo (JP)
(72) Inventor: Erikawa, Akihiko, Kanagawa (JP); Ozawa, Satoshi, Kanagawa (JP); Iida, Takayuki, Kanagawa (JP)
(74) Representative: Klunker . Schmitt-Nilson . Hirsch

(57) **Abstract**

A first irradiation portion that radiates white light onto a subject, a second irradiation portion that radiates narrow bandwidth light having a narrower wavelength bandwidth than the white light, and an observation window used to observe the subject are respectively disposed on a leading end surface of an endoscope insertion portion. Each of the first and second irradiation portions includes a pair of irradiation windows for emitting light therefrom. A straight line passing through a center point of the observation window and bisecting the leading end surface is defined as a boundary line. The pair of irradiation windows of the first irradiation portion are disposed on both sides of the boundary line. The pair of irradiation windows of the second irradiation portion are disposed on the both sides of the boundary line. Spectra of lights radiated from the irradiation windows of the second irradiation portion can be changed individually.

## Description

### BACKGROUND OF THE INVENTION

### 1. Technical Field

The present invention relates to an endoscope apparatus.

### 2. Description of the Related Art

Generally, an endoscope apparatus is configured such that an irradiation window and an observation window are respectively disposed in the leading end of an endoscope insertion portion to be inserted into a body cavity, illumination light is radiated from the irradiation window, and an image of a portion to be observed within the body cavity is captured through the observation window. Then, the thus obtained observation image is displayed on a monitor screen to perform an endoscopic diagnosis for the portion to be observed. Examples of the illumination light include white light emitted from a xenon lamp, a halogen lamp or the like. Recently, in some cases, special light having a specific narrow bandwidth wavelength in combination with the white light is used (see JP 2007-21084 A and JP 2008-259722 A (corresponding to US 2008/0255426 A)). Under the special light radiation, it is possible to carry out various types of observations according to objects: for example, an observation which is made with emphasis on the capillary or mucous membrane fine pattern of a mucous membrane tissue surface layer; an observation which is made through the autofluorescence from the tissues of a living body; and, an observation which is made through the fluorescence of a labeling reagent such as Indo-cyanine Green (ICG) when the labeling reagent is administrated into a living body.

Here, the position of the irradiation window in the leading end of the endoscope insertion portion is set properly in response to the position of the observation window. In the case where the irradiation window is disposed improperly with respect to the observation window, when an area to be observed has unevenness, it cannot be radiated uniformly, with the result that uneven radiation or shadow would appear in the observation image. In view of this, JP 2007-21084 A employs a structure in which multiple irradiation windows are disposed symmetrically with respect to an observation window and around the observation window. Also, JP 2001-166223 A (corresponding to US 2001/0003142 A) proposes a structure in which irradiation windows are disposed on both sides with an observation window being disposed therebetween in order to prevent such uneven radiation.

However, in order to enable various types of special light observations in an endoscope apparatus for making such special light observations, such a structure may be required that multiple types of excitation lights are emitted selectively or simultaneously. In this structure, in the case where multiple irradiation windows are to be disposed in the leading end of the endoscope insertion portion, a space for the irradiation windows might occupy much space, which would be disadvantageous in reducing the diameter of the endoscope insertion portion. On the other hand, it is also considered that the irradiation windows are used in common to reduce the number of irradiation windows used. However, depending on the position relationship between the irradiation windows and observation window, uneven radiation would be easy to occur.

Also, in the special light observations, observation images are obtained by a system which radiates the multiple types of excitation light individually one by one to capture the images of the portion to be observed, or a system which switches light for every imaging frame to obtain the observation images. However, especially, when a moving image is displayed, in some cases, the frame rate might be slowed down and thus the display quality would be deteriorated.

### SUMMARY OF THE INVENTION

Exemplary embodiments of the present invention provide an endoscope apparatus which has two pairs of irradiation windows for illuminating an area to be observed in the leading end of the endoscope insertion portion while the diameter of the endoscope insertion portion can be reduced and uneven radiation can be prevented, and also which can simultaneously radiate narrow bandwidth light respectively having different spectra, whereby multiple-purpose light diagnoses can be realized with high accuracy.

According to an aspect of the invention, an endoscope apparatus includes an endoscope insertion portion, a light source section, and a light source control section. The endoscope insertion portion is configured to be inserted into a subject. The light source section supplies light to the endoscope insertion portion. A first irradiation portion that radiates white light onto the subject, a second irradiation portion that radiates to the subject narrow bandwidth light having a narrower wavelength bandwidth than the white light, and an observation window that is used to observe the subject are disposed on a leading end surface of the endoscope insertion portion. Each of the first and second irradiation portions includes a pair of irradiation windows for emitting lights therefrom. A straight line passing through a center point of the observation window and bisecting the leading end surface of the endoscope insertion portion is defined as a boundary line. The pair of irradiation windows of the first irradiation portion are disposed on the leading end surface on both sides of the boundary line. The pair of irradiation windows of the second irradiation portion are disposed on the leading end surface on the both sides of the boundary line. The light source control section is configured to individually change spectra of the lights radiated from the respective irradiation windows of the second irradiation portion.

With the above endoscope apparatus, the irradiation windows of the first irradiation portion, which radiate the white light, and the irradiation windows of the second irradiation portion, which radiate the narrow bandwidth lights, are disposed at such positions on the leading end surface of the endoscope insertion portion where good space efficiency can be provided and generation of uneven radiation can be prevented. Also, the spectra of the narrow bandwidth lights emitted from the respective irradiation windows of the second irradiation portion can be controlled individually. Thereby, the narrow bandwidth lights having the different spectra can be emitted simultaneously from the respective irradiation windows of the second irradiation portion. Therefore, in addition to a normal observation under white light illumination and a special light observation using the narrow bandwidth light, the narrow bandwidth lights having the different spectra can be radiated. Thereby, multipurpose diagnosis can be realized with high accuracy.

### BRIEF DESCRIPTION OF THE DRAWINGS

Fig. 1 is an schematic block diagram of the configuration of an endoscope apparatus according to an embodiment of the invention.
Fig. 2 is an appearance view of an example of the endoscope apparatus shown in Fig. 1.
Fig. 3A is a sectional configuration view of a light projection unit including a light diffusion member.
Fig. 3B is a sectional configuration view of a light projection unit including a fluorescent member.
Fig. 4 is a graph showing a light emission spectrum of a blue laser light from a laser light source and a light emission spectrum of fluorescence into which a wavelength of the blue laser light is converted by the fluorescent member.
Fig. 5 is a perspective view schematically showing the configuration of an endoscope leading end portion.
Fig. 6 is an exploded view of the endoscope leading end portion shown in Fig. 5.
Fig. 7 is a section view taken along a A-A line shown in Fig. 5.
Fig. 8 is a front view of the endoscope leading end portion when viewed from a B direction shown in Fig. 5.
Fig. 9 is a block diagram of a light source device.
Fig. 10 is a configuration view of an imaging optical system.
Fig. 11 is an explanatory view showing spectral characteristics of the imaging optical system and an illumination optical system.
Fig. 12 is an explanatory view of kinds of light to be emitted from the irradiation windows according to respective irradiation patterns.
Fig. 13 is a timing chart of emitting timings of light from the respective irradiation windows with respect to respective imaging frames.
Fig. 14 is a graph showing spectral characteristics of absorbance of reduced hemoglobin and oxygenated hemoglobin.
Fig. 15 is a graph showing a two dimensional map in which magnitudes of values of S 1/S3 and S2/S3 are expressed on orthogonal two axes.
Fig. 16 is a timing chart of a first control example for switching light emitted from the respective irradiation windows in accordance with the imaging frames.
Fig. 17 is a graph showing spectral profiles of respective laser light having 405 nm, 445 nm and 473 nm in center wavelength and a detection sensitivity of imaging elements B, G and R.
Fig. 18 is a timing chart of a second control example for switching light emitted from the respective irradiation windows in accordance with the imaging frames.
Fig. 19 is a typical view of a surface layer blood vessel and a deep layer blood vessel.
Fig. 20 is an explanatory view of a method for generating a deep layer blood vessel image.
Fig. 21 is a flow chart of a segment extraction process using edge detection.
Fig. 22 is a timing chart of emission timings of light from the respective irradiation windows with respect to the imaging frames.
Fig. 23 is a timing chart of timings of light emission from the respective irradiation windows with respect to the imaging frames.
Fig. 24 is a configuration view of another example of the imaging optical system.
Fig. 25 is a plan view of a leading end surface of the endoscope leading end portion.
Fig. 26 is a schematic section view of the endoscope leading end portion.
Fig. 27 is a graph showing a distribution of light amount ratio among R, G and B detected light with a central pixel value of the captured image as a reference.
Fig. 28 is a schematic configuration view of a modification of the light source device.
Fig. 29A is a partial schematic configuration view of another modification of the light source device.
Fig. 29B is a plan view of a rotation optical filter.
Fig. 30 is a plan view of the leading end surface of the endoscope leading end portion.

### DETAILED DESCRIPTION OF EMBODIMENTS OF THE INVENTION

Now, description will be given below specifically on embodiments of the invention with reference to the accompanying drawings.

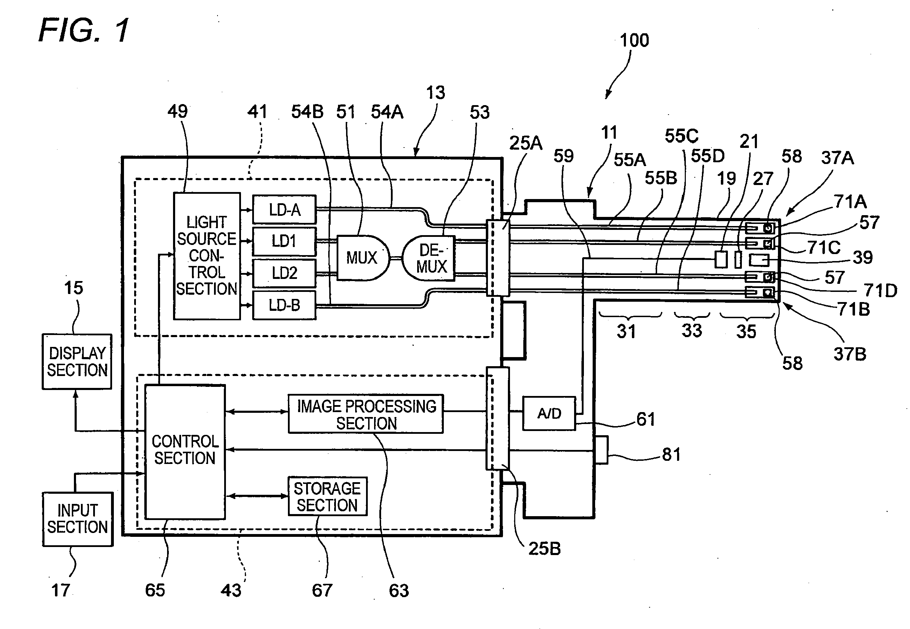

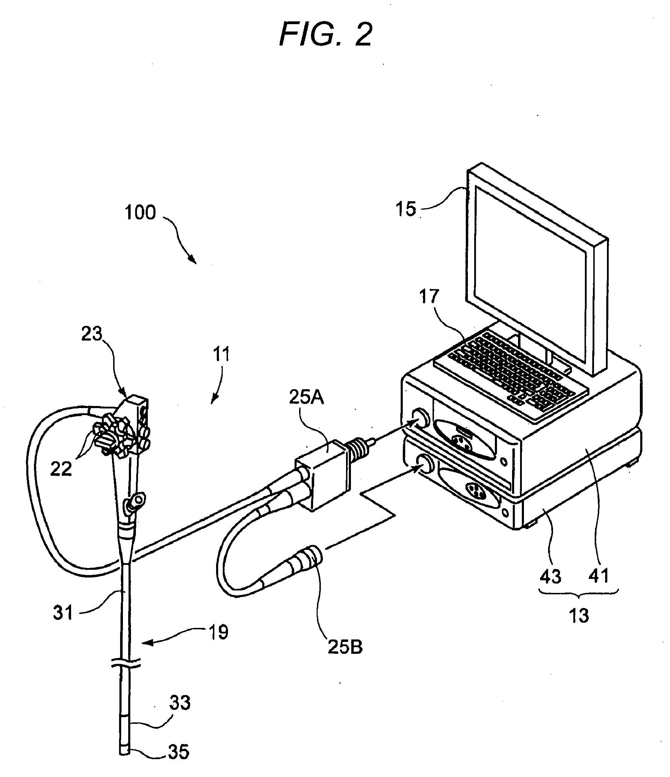

Fig. 1 is an explanatory view a schematic block diagram of the configuration of an endoscope apparatus according to an embodiment of the invention. Fig. 2 is an appearance view of an example of the endoscope apparatus shown in Fig. 1.

As shown in Figs. 1 and 2, an endoscope apparatus 100 includes an endoscope 11, a control apparatus 13 to which the endoscope 11 is connected, a display section 15 for displaying image information and the like thereon, and an input section 17 for accepting an input operation. The endoscope 11 is an electronic endoscope which includes a illumination optical system for emitting illumination light from a leading end of an endoscope insertion portion 19 to be inserted into a subject, and an imaging optical system having an imaging device 21 (see Fig. 1) for capturing an image of an area to be observed.

The endoscope 11 includes the endoscope insertion portion 19, an operation portion 23 (see Fig. 2) for carrying out a bending operation of the leading end of the endoscope insertion portion 19 and an operation for observation, and connector portions 25A, 25B for detachably connecting the endoscope 11 to the control apparatus 13. Although not shown, in interior portions of the operation portion 23 and the endoscope insertion portion 19, there are provided various channels such as a forceps channel into which a tissue collection treatment tool or the like is to be inserted, and a channel for feeding air/water.

The endoscope insertion portion 19 has a flexible soft portion 31, a bending portion 33 and a leading end portion (which may be hereinafter referred to as an endoscope leading end portion) 35. In the endoscope leading end portion 35, as shown in Fig. 1, there are provided irradiation ports 37A, 37B for radiating lights onto the area to be observed, and an imaging device 21 such as a CCD (Charge Coupled Device) image sensor or a CMOS (Complementary Metal-Oxide Semiconductor) image sensor for obtaining image information of the area to be observed. Also, an object lens unit 39 is provided on a light receiving surface side of the imaging device 21.

The bending portion 33 is interposed between the soft portion 31 and the leading end portion 35 and can be freely bendable by rotationally operating an angle knob 22 provided on the operation portion 23 shown in Fig. 2. The bending portion 33 can be bent in an arbitrary direction and at an arbitrary angle according to a portion of a subject for which the endoscope 11 is use, whereby the observing direction of the irradiation ports 37A, 37B of the endoscope leading end portion 35 and the imaging device 21 can be directed to a desired observing portion. Description will be given later in detail on the structure of the irradiation ports 37A, 37B of the endoscope insertion portion 19.

The control apparatus 13 includes a light source device 41 for generating illumination light(s) to be supplied to the irradiation ports 37A, 37B of the endoscope insertion portion 19, and a processor 43 for performing image processing for an image signal transmitted from the imaging device 21. The control apparatus 13 is connected to the endoscope 11 through the connector portions 25A, 25B. Also, the above-mentioned display section 23 and input section 17 are connected to the processor 43. The processor 43 performs image processing for an imaging signal transmitted from the endoscope 11 based on an instruction from the operation portion 23 and input section 17 of the endoscope 11 to generate a display image, and supplies the thus generated display image to the display section 15.

The light source device 41 includes multiple kinds of laser light sources the center light emission wavelengths of which are different from each other. In this structure example, as shown in Fig. 1, the light source device 41 includes a laser light source LD1 having a center light emission wavelength of 405 nm, a laser light source LD2 having a center light emission wavelength of 445 nm, and a first laser light source section LD-A and a second laser light source section LD-B which respectively includes multiple kinds of laser light sources details of which will be given later.

The lights of the respective laser light sources LD1, LD2 and the first and second laser light source sections LD-A, LD-B are controlled individually by a light source control section 49. The respective laser lights can be generated individually or simultaneously. That is, the emission timings of the respective laser lights and the emission light amount ratio thereof can be changed arbitrarily. Thus, the spectra of lights emitted from the irradiation windows from which the respective laser lights are emitted can be changed individually.

LD1 is a light source which emits violet laser light having a center wavelength of 405 nm, while LD2 is a light source for normal observation which emits blue laser light having a center wavelength of 445 nm and generates white illumination light using a fluorescent member serving as a wavelength converting member (which will be described later). Examples of laser light sources mounted on the laser light sources LD1, LD2 and the first and second laser light source sections LD-A, LD-B include an InGaN-based broad area type laser diode, an INGaNAs-based laser diode, and a GaNAs-based laser diode.

Laser lights, which are emitted from the respective laser light sources, are respectively introduced into an optical fiber by a condenser lens (not shown). The laser lights from LD1 and LD2 are combined together by a combiner 51 and are then divided into two lights by a coupler 53. Then, the lights are transmitted to the connector portion 25A. Thus, the laser lights from LD1 and LD2 can be transmitted evenly to two systems of optical paths with their speckles reduced. Alternatively, a simplified structure may be employed in which, the laser lights from LD1 and LD2 are transmitted directly to the connector portion 25A without using the combiner 51 nor the coupler 53.

Also, the laser light from the first laser light source section LD-A are transmitted through an optical fiber 54A to the connector portion 25A, and the laser light from the second lager light source portion LD-B are transmitted through an optical fiber 54B to the connector portion 25A.

The respective laser lights transmitted to the connector portion 25A are then introduced into optical fibers 55A to 55D which respectively extend from the connector portion 25A to the endoscope leading end portion 35. Specifically, the laser lights from the laser light sources LD1 and LD2 are guided through their associated optical fibers 55B and 55C to the endoscope leading end portion 35. The laser light from the first laser light source section LD-A and the laser light from the second laser light source section LD-B are guided to the endoscope leading end portion 35 through their associated optical fiber 55A and optical fiber 55D, respectively.

Light projection units 71A, 71B, 71C and 71D are arranged on the endoscope leading end portion 35. The optical fiber 55A is connected to the light projection unit 71A. The optical fiber 55 is connected to the light projection unit 71B. The optical fibers 55B and 55C are connected to the light projection units 71C and 71D, respectively.

The light projection unit 71A includes the optical fiber 55A and a light diffusion member 58 and is configured such that light emitted from a light emission end of the optical fiber 55 is diffused by the light diffusion member 58 and is radiated ahead of the optical path. The light projection unit 71B includes the optical fiber 55D and a light diffusion member 58 and is configured similarly to the light projection unit 71A.

The light projection unit 71C includes an optical fiber 55B and a fluorescent member 57 and is configured such that light emitted from a light emission end of the optical fiber 55B is used to excite the fluorescent member 57 to generate light and that the generated light is radiated ahead of the optical path together with the light emitted from the optical fiber 55B. The light projection unit 71C includes the optical fiber 55C and a fluorescent member 57 and is configured similarly to the light projection unit 71C.

The paired light projection units 71A and 71B and the paired light projection units 71C and 71D are respectively disposed on both sides of the object lens unit 39 with sandwiching the object lens unit 39 serving as the observation window of the endoscope leading end portion 35. White lights are emitted from the pair of light projection units 71C and 71D (an example of a first irradiation portion). Laser lights which are respectively emitted from the first laser light source section LD-A and second laser light source section LD-B and which serve as special light are emitted from the pair of projection units 71A and 71B (an example of a second irradiation portion).

Here, the optical fibers 55A to 55D are multi-mode fibers. For example, there can be used a small-diameter fiber cable having a core diameter of 105 µm, a clad diameter of 125 µm, and a diameter including a protection layer serving as a coating of φ 0.3 mm to 0.5 mm.

Next, description will be given below on the specific structure of the light projection units 71A to 71D.

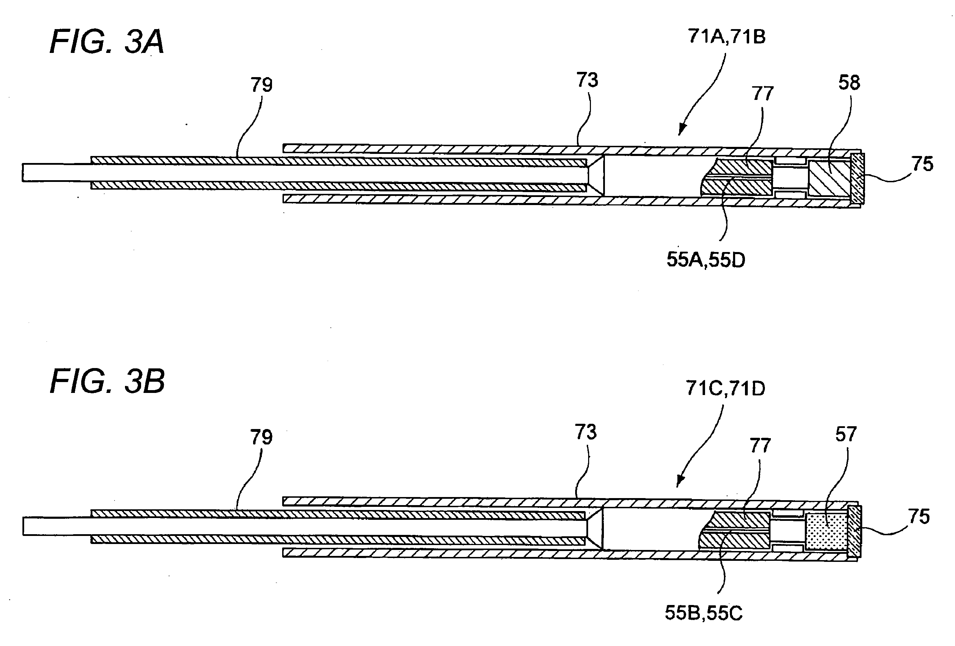

Fig. 3A is a sectional configuration view of the light projection units 71A and 71B. Fig. 3B is a sectional configuration view of the light projection units 71C and 71D. The light projection units 71A and 71B are the same in structure. Each of the light projection units 71A and 71B includes the light diffusion member 58, a cylindrical sleeve member 73 for covering the outer periphery of the light diffusion member 58, a protection glass (irradiation window) 75 for sealing one end side of the sleeve member 73, and a ferrule 77 which is inserted into the sleeve member 73 and is used to hold the optical fiber 55A (55D) on the center axis thereof. Also, a flexible sleeve 79 is inserted between a portion of the optical fiber 55A (55D) which extends from a rear end side of the ferrule 77 with being covered with an outer sheath and the sleeve member 73. The flexible sleeve 79 covers the outside of the outer sheath.

On the other hand, the light projection units 71C and 71D are the same in structure and are also the same in structure as the light projection units 71A and 71B except that, instead of the light diffusion members 58 of the light projection units 71A and 71B, the fluorescent members 57 are provided and lights are introduced from the optical fibers 55B and 55C.

The fluorescent members 57 of the light projection units 71C and 71D include multiple kinds of fluorescent materials (for example, YAG based fluorescent material or BAM (BaMgAl₁₀ O₁₇)) which absorb a portion of the blue laser light from the laser light source LD2 and are excited by such blue laser light to thereby emit green to yellow light. With this configuration, the emitted green to yellow light, which is generated with the blue laser light as the excitation light, and blue laser light transmitting without being absorbed by the fluorescent members 57 are combined together to thereby generate white (pseudo-white) illumination light.

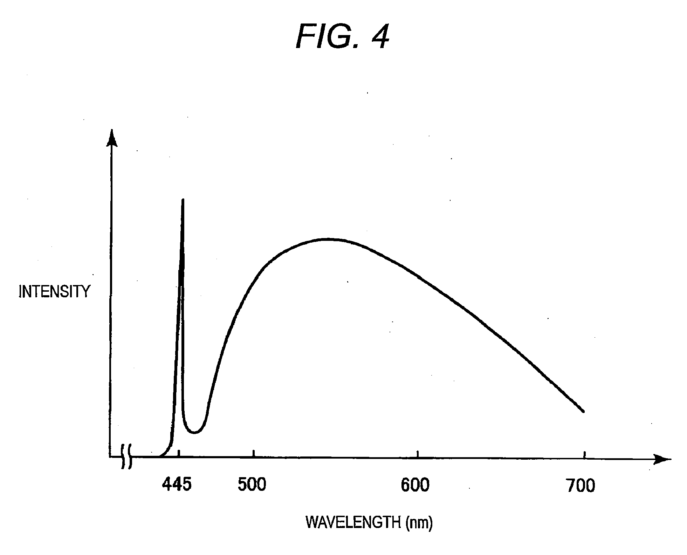

Fig. 4 is a graph showing a light emission spectrum of the blue laser light from the laser light source LD2 and a light emission spectrum after the wavelength of such blue laser light is converted by the fluorescent member 57. The blue laser light is expressed by an emission line having a center wavelength of 445 nm, while the light generated from the fluorescent member 57 by the blue laser light provides a spectral intensity distribution in which emission intensity increases in the wavelength band of substantially 450 nm to 700 nm. The profile of the emitted light and the blue laser light constitute the white light. As in this structure example, when the laser light source is used as the excitation light source of the fluorescent member, white light having high emission efficiency and high intensity can be obtained. Also, not only the intensity of the white light can be adjusted easily but also variations in the color temperature and chromaticity of the white light can be controlled to a minimum.

Here, the term "white light" used in the specification is not limited to the white light that strictly contains all wavelength components of a visible wavelength band, but it may be any light so long as it includes light in a specific wavelength band, for example, reference color light such as R (red), G (green) and B (blue). For example, the "white light" may include, in a wide sense, light containing wavelength components ranging from green to red or light containing wavelength components ranging from blue to green.

The fluorescent member 57 can prevent superimposition of a noise interfering with imaging due to a speckle caused by the coherence of a laser light and prevent a moving image from flickering when it is displayed. Also, the fluorescent member 57 is preferably formed of such a material that particle diameters of fluorescent materials and filler are selected considering a refractive index difference between the fluorescent materials constituting a phosphor and a fixing/curing resin serving as the filler. Also, the fluorescent member 57 is preferably formed of such a material that the material less absorbs red light and infrared light and largely scatter red light and infrared light. In this case, the diffusion effect of red light and infrared light can be enhanced without degrading the intensity of light, thereby being able to reduce the optical loss of the fluorescent member 57.

The light diffusion members 58 of the light projection units 71A and 71B are made of light transmittable resin material which allows the laser lights from the first and second laser light source sections LD-A and LD-B to pass therethrough. Instead of the light transmittable resin material, light transmittable ceramics or glass may be used. Also, the light diffusion member 58 may also have a structure that, in the surface or intermediate layer thereof, there is provided a light diffusion layer including minute uneven portions or mixed with particles (fillers or the like) respectively having different refractive indexes. Or, there may also be used translucent material. Thus, the light emitted from the light diffusion member 58 is narrow wavelength bandwidth light the light amount of which is uniform in a given radiation area due to the deflection and diffusion of the light.

Here, referring back again to Fig. 1, the white light, which is generated by the blue laser light and the lights emitted from the fluorescent members 57 as described above, and the narrow wavelength bandwidth light of a narrow spectrum emitted from the respective laser light are radiated from the endoscope leading end portion 35 toward the area to be observed of the subject. When the illumination light is projected onto the area to be observed, the state of the area to be observed is captured by the imaging device 21 through the object lens unit 39 after unnecessary light component is removed by an optical filter 27 which will be described later.

An image signal of the captured image, which is output from the imaging device 21 after the capturing is transmitted through a scope cable 59 to an A/D converter 61, where it is converted into a digital signal, and this digital signal is then input through the connector portion 25B to the image processing section 63 of the processor 43. The image processing section 63 executes various processes such as a white balance correction process, a gamma correction process, a contour-emphasis process, and a color correction process for the image signal transmitted from the imaging device 21 which has been converted into the digital signal. The captured image signal processed by the image processing section 63 is formed as an endoscope observation image together with various kinds of information by a control section 65, and is then displayed on the display section 15. Also, the captured image signal may be stored in a storage section 67 having a memory and a storage device.

Next, description will be given below specifically on the structure of the endoscope leading end portion.

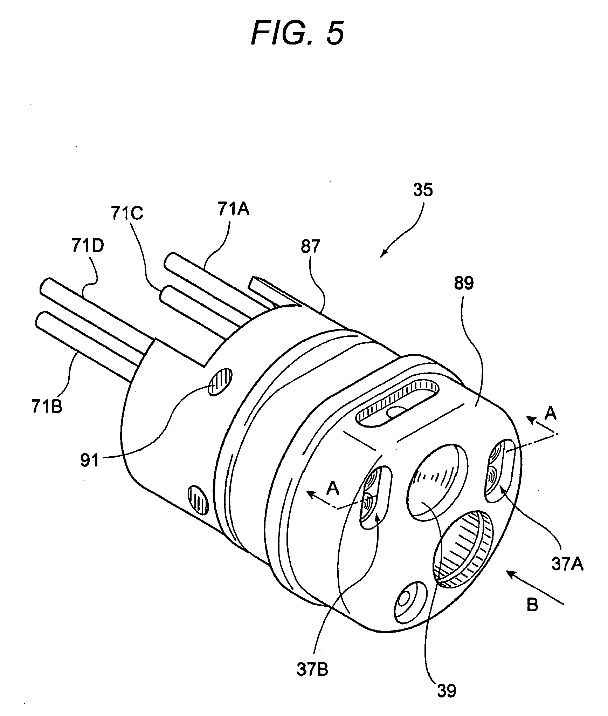

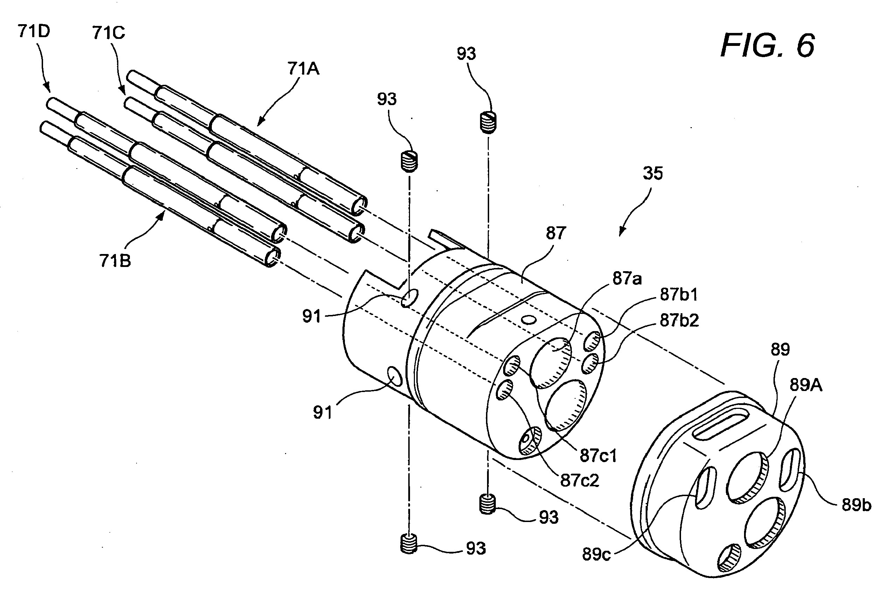

Fig. 5 is a perspective view of the schematic structure of the endoscope leading end portion. Fig. 6 is an exploded view of the endoscope leading end portion shown in Fig. 5.

As shown in Figs. 5 and 6, in the endoscope leading portion 35, the above-mentioned light projection units 71A to 71D are mounted into a leading end hard portion 87 made of stainless steel or the like and formed with plural though holes along the longitudinal direction thereof. The leading end hard portion 87 is formed with a through hole 87a into which the imaging optical system including the imaging device 21 shown in Fig. 1 is stored. Through holes 87b1, 87b2, and through holes 87c1, 87c2 are formed on both sides of the leading end hard portion 87 with the through hole 87a being its center. The light projection units 71A, 71C are inserted into the holes 87b1, 87b2, respectively. The light projection units 71D, 71B are inserted into the holes 87c1, 87c2, respectively.

Also, the leading end side of the leading end hard portion 87 is covered with a leading end rubber cap 89. The outer periphery of the leading end hard portion 87 is covered with a outer sheath tube (not shown). The leading end rubber cap 89 is formed with through holes 89a, 89b, 89c, which correspond to the respective through holes 87a, 87b1, 87b2, 87c1, 87c2, of the leading end hard portion 87. With this configuration, the observation window to be used by the object lens unit 39 and the irradiation ports 37A, 37B for the light projection units 71A to 71D are allowed to open there.

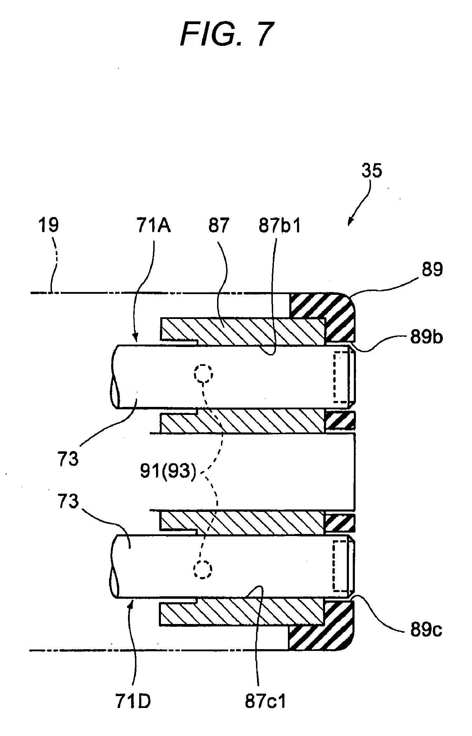

Fig. 7 shows a section view taken along a A-A line shown in Fig. 5. After the light projection units 71A, 71C are respectively inserted into their associated through holes 87b1, 87c1 of the leading end hard portion 87, by fastening the light projection units 71D, 71B through fixing holes 91 (see Figs. 4 and 5) communicating with the through holes 87b1, 87c1 using screws (work screws) 93, the light projection units 71D, 71B are fixed to the leading end hard portion 87. Also, the light projection units 71B, 71D are fixed to the leading end hard portion 87 similarly by fastening the light projection units 71B, 71D using screws 93.

With the structure of the endoscope including the above-mentioned light projection units 71A to 71D, since the light projection units 71A to 71D are detachably fixed to the leading end hard portion 87 by the screws 93 in such a state where the light projection units 71A to 71D are inserted into the through holes 87b1, 87b2, 87c1, 87c2 of the leading end hard portion 87, the light projection units 71A to 71D are easy to replace. Thereby, the maintenance property of the endoscope can be enhanced. That is, when the illumination light intensity is reduced or a color tone is varied due to long use of the endoscope, the currently used light projection units can be simply replaced with new light projection units.

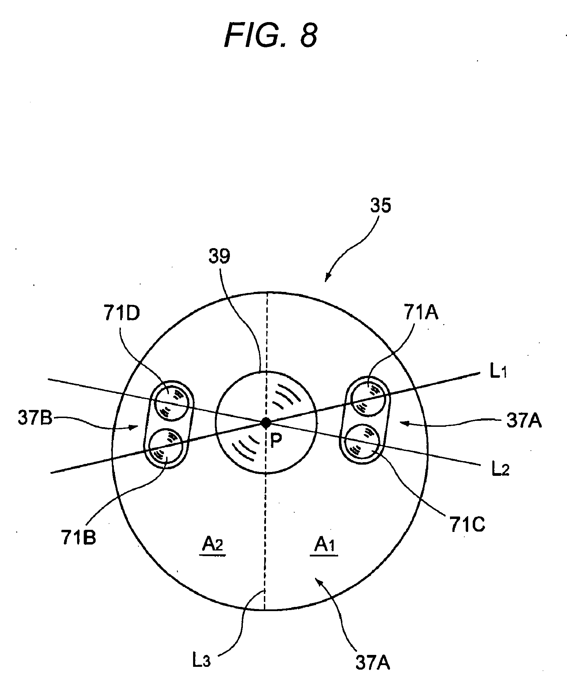

Fig. 8 is a front view of the endoscope leading end portion when viewed from a B direction shown in Fig. 5. As described above, the light projection units 71A to 71D are disposed on the both sides of the object lens unit 39 so that the light projection units 71A and 71C can radiate light from the irradiation port 37A and the light projection units 71B and 71D can radiate light from the irradiation port 37B. The pair of light projection units 71A and 71B including the light diffusion members 58 (see Fig. 3A) are disposed so that a line L1 connecting positions of the protection glasses 75 (see Fig. 3) serving as the irradiation windows crosses a lens area of the object lens unit 39 serving as the observation window. Also, the pair of the light projection units 71B, 71C including the fluorescent members 57 (see Fig. 3B) are disposed so that a line L2 connecting positions of the protection glasses 75 (see Fig. 3B) crosses the lens area of the object lens unit 39.

That is, the plural irradiation windows include a first irradiation portion (light projection units 71C, 71D) having a pair of irradiation windows for radiating white light through the fluorescent members 57, and a second irradiation portion (light projection units 71 A, 7 1 B) having a pair of irradiation windows for radiating a narrow bandwidth light having a narrower wavelength band than the white light. Assuming that a boundary line L3 denotes a line passing through a center point P of the observation window and bisecting a leading end surface 35a of the endoscope leading end portion 35, the pair of irradiation windows of the first irradiation portion are disposed on the leading end surface 35a on both sides of the boundary line L3, and the pair of irradiation windows of the second irradiation portion are disposed on the leading end surface 35a on the both sides of the boundary line L3.

In other words, the irradiation windows (71C, 71D) of the first irradiation portion are disposed within the leading end surface areas A1 and A2, which are bisected by the boundary line L3, respectively, and the irradiation windows (71A, 71B) of the second irradiation portion are disposed within the leading end surface areas A1 and A2, which are bisected by the boundary line L3, respectively.

With this structure example, the respective light projection units 71A to 71D are disposed in a space efficiency enhanced state so that the point of intersection between the lines L1 and L2 exists in the lens area of the object lens unit 39. The light projection units 71C, 71D for projecting the white illumination light are disposed on the both sides of the object lens unit 39 of the endoscope leading end portion 35. Thereby, the light projection units 71C, 71D can project the white light evenly from the both sides of the object lens unit 39 and can prevent occurrence of uneven radiation.

Next, description will be given below on the specific structures of the first and second laser light source sections LD-A, LD-B and the structure of the light source device 41 including a combination of these laser light source sections LD-A, LD-B.

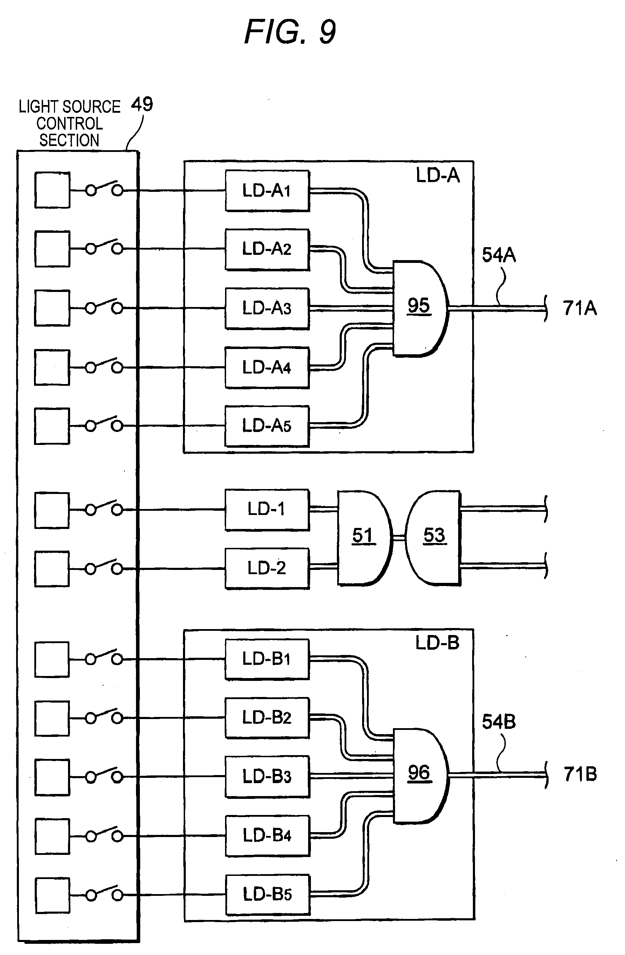

Fig. 9 shows a block diagram of the light source device 41. As shown in Fig. 9, the first and second laser light source sections LD-A and LD-B are the same in structure, while the first and second laser light source sections LD-A and LD-B respectively include plural laser light sources having different center light emission wavelengths. Table 1 shows the light source kinds of the first and second laser light source sections LD-A and LD-B.

**[Table 1]**

| First laser light source section | Second laser light source section | Center light emission wavelength |
|---|---|---|
| LD-A1 | LD-B1 | 375 nm |
| LD-A2 | LD-B2 | 405 nm |
| LD-A3 | LD-B3 | 445 nm |
| LD-A4 | LD-B4 | 473 nm |
| LD-A5 | LD-B5 | 780 nm |

The light source control section 49 controls lighting timings and emission light intensities of the respective laser light sources LD-A1 to A5, LD-B1 to B5 and LD1, LD2. By combining the laser light sources, arbitrary illumination light can be generated. The laser lights emitted from the laser light sources LD-A1 to A5 are combined together by a combiner 95 and are then output to the optical fiber 54A. Also, similarly, the laser lights emitted from the laser light sources LD-B1 to B5 are combined together by a combiner 96 and are then output to the optical fiber 54B. Here, the center light emission wavelengths of the respective laser light sources used may be in the range of ± 10 nm of the respective center light emission wavelengths shown in Table 1. This applies similarly to the light sources LD1 and LD2. Also, the emission light wavelengths can be set properly according to the objects or kinds of special light observations to be carried out.

With the above configuration, white light is emitted from the light projection units 71C, 71D, and narrow bandwidth light of any of the wavelengths shown in Table 1 is (are) emitted from the light projection units 71A, 71B in such a manner that narrow bandwidth lights are combined together arbitrarily.

Next, description will be given below on the imaging optical system.

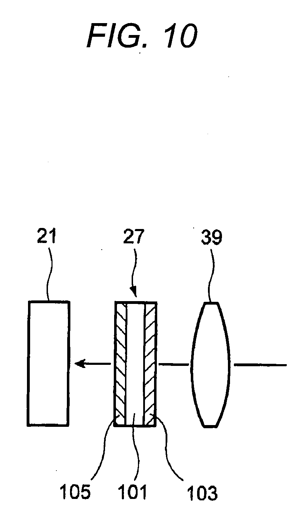

As shown in Fig. 10, the imaging optical system includes the imaging device 21 for capturing an image of an observation area, the object lens unit 39, and an optical filter 27 interposed between the imaging device 21 and the object lens unit 39. The optical filter 27 is configured in the following manner. That is, a near infrared light cutting filter 103 for removing components of near infrared light (wavelength of 780 nm) (details of which will be discussed later) transmitted from the laser light sources LD-A5, LD-B5 is bonded to on one surface of a transparent optical substrate 101 of the optical filter 27. Also, an ultraviolet light cutting filter 105 for removing components of ultraviolet light (wavelength of 375 nm) (details of which will be discussed later) transmitted from the laser light sources LD-A1, LD-B1 are bonded to the other surface of the transparent optical substrate 101 of the optical filter 27.

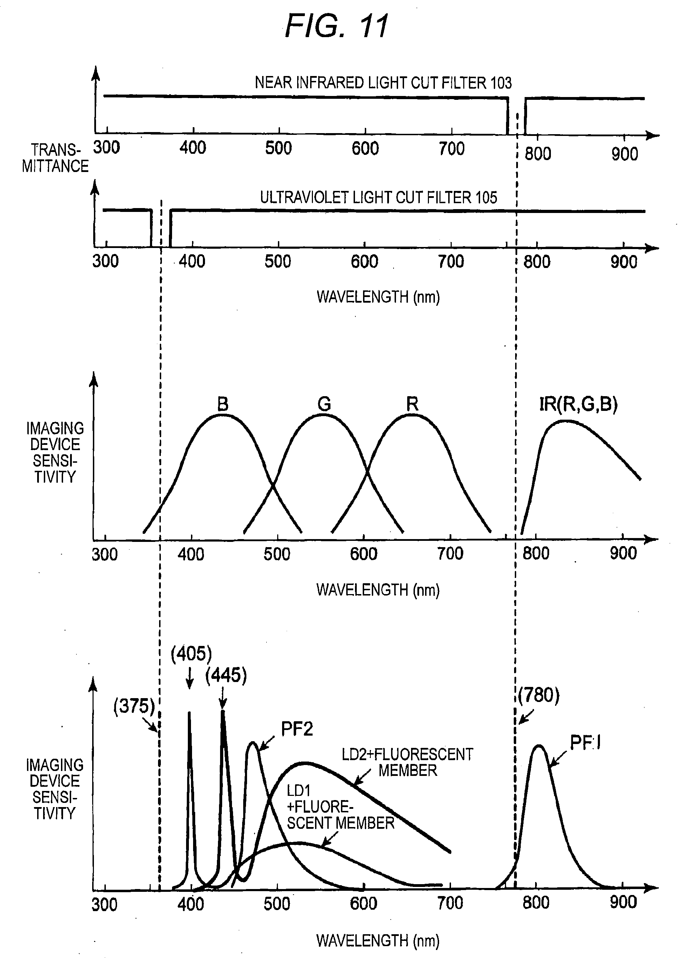

Fig. 11 shows the spectral characteristics of the imaging optical system and the illumination optical system. As shown in Fig. 11, the imaging device 21 is a two-dimensional image sensor including a large number of light receiving pixels having sensitivities to B, G and R light. B, G and R color filters corresponding to the respective pixels are provided above the image pickup surface of the imaging device 21 in the Bayer arrangement. Image information of each color is output. The respective light receiving pixels also have sensitivity to near infrared light as well. Therefore, the imaging device 21 can detect image information with respect to the B light, G light, R light and near infrared light. The imaging device 21 is a primary color system imaging device having sensitivities to the basic colors of B, G and R. Alternatively, the imaging device 21 may be a complementary color system imaging device having sensitivities to the basic colors of cyan C, magenta M and yellow Y (and G). In this case, by converting C, M and Y to B, G and R, this imaging device can be treated similarly to the primary color system imaging device. The arrangement of the color filters of the imaging device 21 may not be limited to the Bayer arrangement. Another arrangement may be adopted in which color filters of other colors are combined.

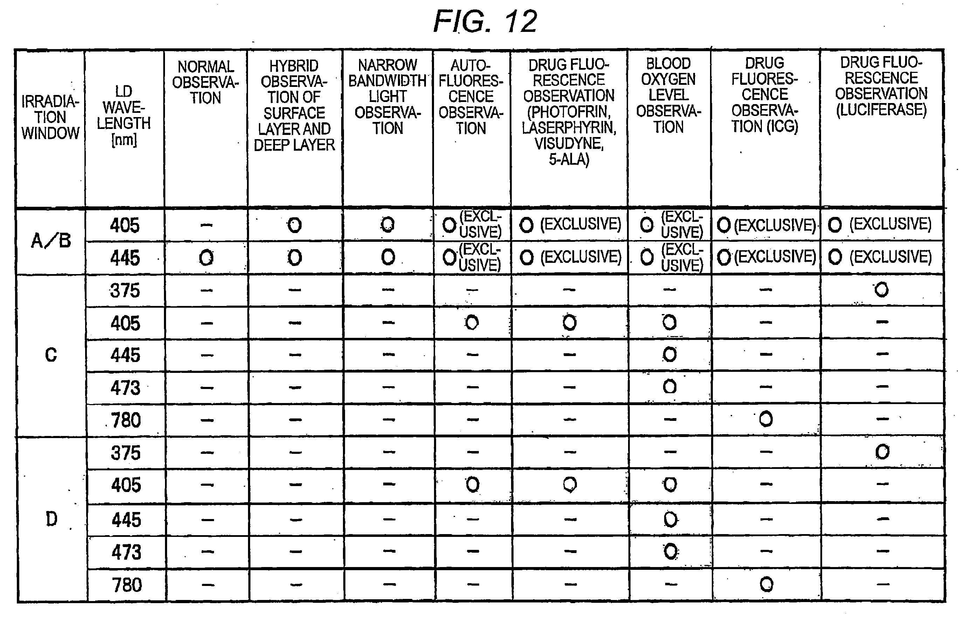

Next, description will be given below on various irradiation patterns in which the respective laser lights of the laser light sources LD1, LD2 and the first and second laser source portions LD-A, LD-B are combined in response to an observation mode and the thus combined laser lights are then emitted from the light projection units 71A to 71D. Fig. 12 shows examples of the respective irradiation patterns. In Fig. 12, irradiation windows A, B, C and D respectively represent the irradiation windows of the light projection units 71A, 71B, 71C and 71D. The respective irradiation windows of the light projection units 71A, 71B, 71C and 71D may be hereinafter referred to as irradiation windows A, B, C and D, respectively.

### <First Irradiation Pattern>

The first irradiation pattern is a white illumination pattern in a normal observation time. The light source control section 49 causes the laser light source LD2 to emit the laser light having the center wavelength of 445 nm and causes the white light to be emitted from the irradiation windows C and D. The light source control section 49 turns off output of the other remaining laser light sources to thereby stop light emission of the other remaining laser light sources.

As described above, since the white light are emitted simultaneously from the both of the irradiation windows C and D, even if there exists any uneven portion in an observation portion, uneven radiation and shadow can be prevented. Thereby, a normal observation can be carried out under the uniform illumination light.

### <Second Irradiation Pattern>

The second irradiation pattern is an irradiation pattern in which, in addition to the normal observation under the white illumination, it becomes possible to observe information of a surface layer of tissues.

The light source control section 49 causes the laser light source LD1 to emit the laser light having the center wavelength of 405 nm, causes the laser light source LD2 to emit the laser light having the center wavelength of 445 nm, and causes the white light and the laser light of the laser light source LD1 to be emitted from the irradiation windows C and D. The emission light amount ratio between LD1 and LD2 may be set so that, for example, LD1:LD2 is 1:4. Also, the outputs of the other remaining laser light sources are turned off to thereby stop the light emission thereof.

Since the light amount of LD2 is larger than that of LD1, a distant view can be observed more brightly and information of the tissue surface layer can also be observed.

As to the radiation timings of LD1 and LD2, the radiation using the white light and the radiation using the narrow bandwidth light (the laser light of LD1) may be carried out alternately, or radiating operations using both of the white light and the narrow bandwidth light may be carried out simultaneously.

With this irradiation pattern, in addition to the white light, the narrow bandwidth light having a short wavelength is radiated. Therefore, even in the normal observation time, in addition to an observation image in which a distant view is made brighter, an image component in which blood capillaries of mucous membrane tissue surface layer are emphasized can be obtained.

### <Third Irradiation Pattern>

The third irradiation pattern is an irradiation pattern in which an observation is made in such a manner that, especially, blood vessels of a tissue surface layer and mucous membrane fine patterns are displayed in an emphasized manner.

The light source control section 49 causes the laser light source LD1 to emit the laser light having the center wavelength of 405 nm, causes the laser light source LD2 to emit the laser light having the center wavelength of 445 nm, and causes the white light and the laser light (narrow bandwidth light) of LD1 to be emitted from the irradiation windows C and D. The emission light amount ratio of LD1 to LD2 may be set so that, for example, LD1:LD2 is 7:1. Also, the light source control section 49 turns off ¥outputs of the remaining laser light sources to thereby stop the light emission of the remaining laser light sources.

Since the light amount of LD1 is larger than that of LD2, in the near view observation, a further detailed observation of the tissue surface layer becomes possible.

With this irradiation pattern, an image component in which fine structures of capillary vessels of mucous tissue surface layer and fine patterns of the mucous tissues are emphasized can be obtained. In this narrow bandwidth light observation, it is possible to easily confirm information of the tissue surface layer which cannot be obtained in the normal observation.

Since the light emission amount of LD1 is larger than that of LD2, an observation image in which the surface layer blood vessels are emphasized further can be obtained. Also, since the light emission ratio of LD1 to LD2 can be changed arbitrarily, a distribution of surface layer blood vessels in its depth direction can be observed. Further, since the laser lights from LD1 and LD2 are both emitted from the same irradiation windows A and B, when the observed image is operationally processed, the radiation conditions of the both laser lights can be made to coincide with each other with high accuracy. As a result, variations in the observation image due to differences between the illumination lights can be extracted accurately.

The light emission amount ratio between LD 1 and LD2 can be changed at an arbitrary timing or at a programmed given timing by operating a change-over switch 81 (shown in Fig. 1) provided on the endoscope 11, through an operation from the input section 17, or by the light source device 41. Also, when there is employed a structure in which a previously preset light emission amount ratio can be set through a switch operation or the like, the normal observation image and the tissue surface layer emphasized image can be simply switched over to each other, thereby being able to reduce burden of the endoscope diagnosis operation.

### <Fourth Irradiation Pattern>

The fourth irradiation pattern is an irradiation pattern for carrying out a fluorescent observation.

The light source control section 49 causes one or both of the laser light sources LD1 and LD2 to emit the laser light having the center wavelength of 405 nm and/or the laser light having the center wavelength of 445 nm, and causes the white light or the white light and the laser light (narrow bandwidth light) of LD1 to be emitted from the irradiation windows C, D. Also, the light source control section 49 causes LD-A2 and LD-B2 to emit the laser lights having the center wavelength of 405 nm, and causes these laser lights to be emitted from the irradiation windows A and B. The light source control section 49 turns off outputs of the remaining laser light sources to thereby stop light emission thereof.

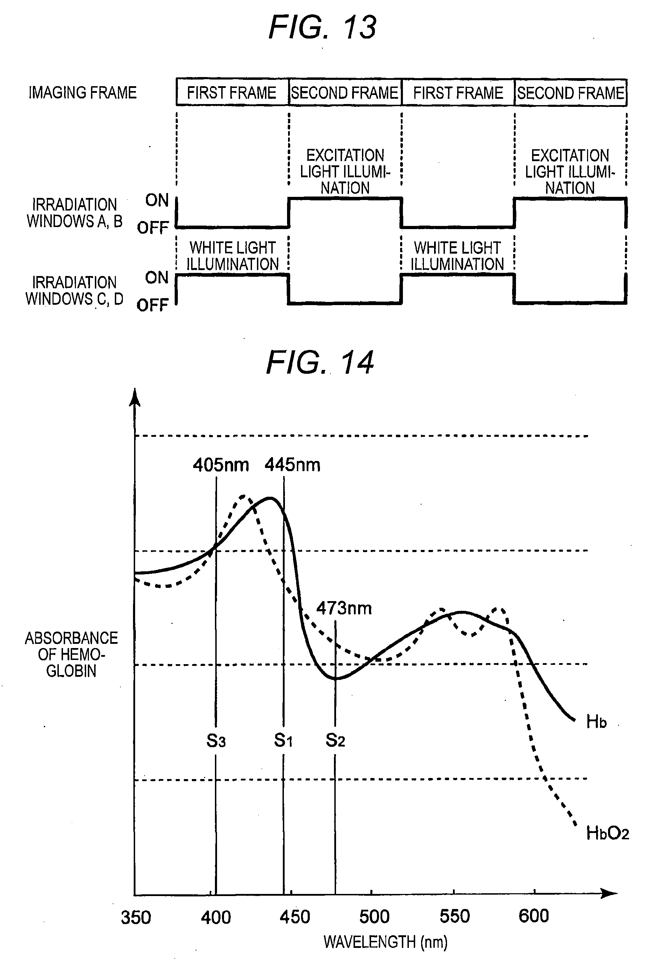

As shown in Fig. 13, the timings of lights to be emitted from the irradiation windows A, B and the irradiation windows C, D are set in synchronous with the imaging frames of the imaging device 21. The lights are emitted alternately every imaging frame but are not emitted simultaneously. That is, a first frame in which the white light or the white light and the narrow bandwidth light are emitted from the irradiation windows C and D for capturing an image and a second frame in which the excitation lights having the wavelength of 405 nm are radiated from the irradiation windows A and B for capturing an image are repeated. A proper image processing is performed for the respective frame images, and the resultant image of each frame is displayed on the display section 15 (see Fig. 1). Alternatively, the respective frame images may be synthesized and displayed.

The first frame is an image for confirming an observation position, while the second frame is a diagnosis image for observing autofluorescence and drug fluorescence. Further frame(s) may be added in addition to the first and second frames.

The excitation lights emitted from the irradiation windows A and B are respectively used to obtain autofluorescence from a living body. Examples of a material that produces the autofluorescence include nicotinamide adenine dinucleotide (NADH) and collagen. NADH is contained abundantly in the living tissues of epithelia in a colon. It has been known that, in lesion portions of the living tissues (for example, tumors or cancers), NADH tends to reduce. Therefore, by radiating excitation light onto a subject to observe the autofluorescence thereof, the lesion portions of the living tissues can be determined based on the fact that the autofluorescence from NADH have weakened.

Also, collagen is contained abundantly in the living tissues of the mucous membrane lower layer such as skins and connective tissues. It has also been known that, in lesion portions of the living tissues, the mucous membrane thereof tends to increase in thickness. If the mucous membrane thickens in this manner, the excitation light is hard to reach the mucous lower layer. Therefore, when the excitation light is radiated onto the subject, the autofluorescence from collagen in the lesion portions of the biogenic tissue are also weaken. By making use of this fact, the lesion portions of the living tissues can be discovered and diagnosed.

Also, the excitation lights emitted from the irradiation windows A and B are used to obtain drug fluorescence from a fluorescent drug introduced into the living body. Examples of the fluorescent drug include Photofrin, Laserphyrin, Visudyne, and 5-ALA (amino-levulinic acid). When any one of these fluorescent drugs is used, it can be excited by a laser light having a center wavelength of 405 nm to generate fluorescence. The fluorescence of 5-ALA is generated due to accumulation of protoporphyrin IX and, as the lesions progress, the wavelength ratio of fluorescence varies.

**[Table 2]**

| Name of Medicines | Excitation wavelength | Emission wavelength |
|---|---|---|
| Photofrin | 405 nm | 660 nm |
| Laserphyrin | 405 nm | 660 nm |
| Visudyle | 405 nm | 660 nm |
| 5-ALA | 405 nm | 635/670 nm |

With this irradiation pattern, when the excitation lights are radiated evenly from the irradiation windows A, B onto the subject, the autofluorescence and drug fluorescence from the entire captured image screen can be observed. Also, since the respective frame images are displayed together, the observation can be carried out while comparing the observation image under the white light illumination with the fluorescence observation image. Thereby, more accurate diagnosis is possible.

Also, with regard to the narrow bandwidth lights emitted from the irradiation windows A and B, the laser lights from LD-A2 and LD-B2 are emitted through the light diffusion members 58 (see Fig. 3A) and thus do not pass through the fluorescent members. This prevents that the light emission components of the fluorescent members is mixed with the fluorescence from the living tissues and appears as noises in the observation image. Also, there is also eliminated the possibility that the emission light intensity is lowered due to the light absorption or light diffusion action of the fluorescent members.

Here, in the above example, the emission lights from the respective irradiation windows are switched every imaging frame. However, this switching operation may be carried out at an arbitrary timing or at a programmed given timing by operating the change-over switch 81 of the endoscope 11 shown in Fig. 1, through an operation from the input section 17, or by the light source device 41.

### <Fifth Irradiation Pattern>

The fifth irradiation pattern is an irradiation pattern for detecting blood oxygen saturation and blood vessel depth. The light source control section 49 causes one or both of the laser light sources LD1 and LD2 to emit the laser light having the center wavelength of 405 nm and/or the laser light having the center wavelength of 445 nm, and causes the white light or the white light and the laser light (narrow bandwidth light) of LD1 to be emitted from the irradiation windows C and D. Also, the light source control section 49 selectively causes one or more of the laser light (center wavelength of 405 nm) of LD-A2, the laser light (center wavelength of 445 nm) of LD-A3, the laser light (center wavelength of 473 nm) of LD-A4, the laser light (center wavelength of 405 nm) of LD-B2, the laser light (center wavelength of 445 nm) of LD-B3 and the laser light (center wavelength of 473 nm) of LD-B4 to be emitted. Then, the control section 49 causes the thus selected laser lights to be emitted from the irradiation windows A and B. The control section 49 turns off outputs of the remaining laser light sources to thereby stop emission of respective laser lights of the remaining laser light sources.

The lights emitted from the irradiation windows C and D are used for white illumination, whereas the lights emitted from the irradiation windows A and B are used as illumination lights to detect blood oxygen saturation and blood vessel depth.

As shown in Fig. 13, the timings of light emission from the irradiation windows A, B and the irradiation windows C, D are set alternately every imaging frame. Thereby, the lights are not radiated from the radiations windows A, B and C, D at the same time but the emission of the lights is switched every imaging frame. That is, the irradiation windows A, B and the irradiation windows C, D emit their lights exclusively.

According to this irradiation pattern, the oxygen saturation and blood vessel depth in the observation area can be found using an absorption spectrum difference between oxygenated hemoglobin HbO₂ and reduced hemoglobin Hb having released oxygen in hemoglobin contained in the erythrocyte of blood. Fig. 14 shows the spectrum characteristics of the absorbance of the oxygenated hemoglobin HbO₂ and the reduced hemoglobin Hb. As shown in Fig. 14, in the vicinity of the wavelength 445 nm, the reduced hemoglobin Hb is higher in absorbance than the oxygenated hemoglobin HbO₂; whereas, in the vicinity of the wavelength 473 nm, the oxygenated hemoglobin HbO₂ is higher in absorbance than the reduced hemoglobin Hb. Also, the laser light has such characteristics that the shorter the wavelength of the laser light is, the shallower the reaching depth of the laser light from the mucous membrane surface layer is. Therefore, the reaching depth of the laser light becomes deeper in order of wavelengths 405 nm, 445 nm and 473 nm.

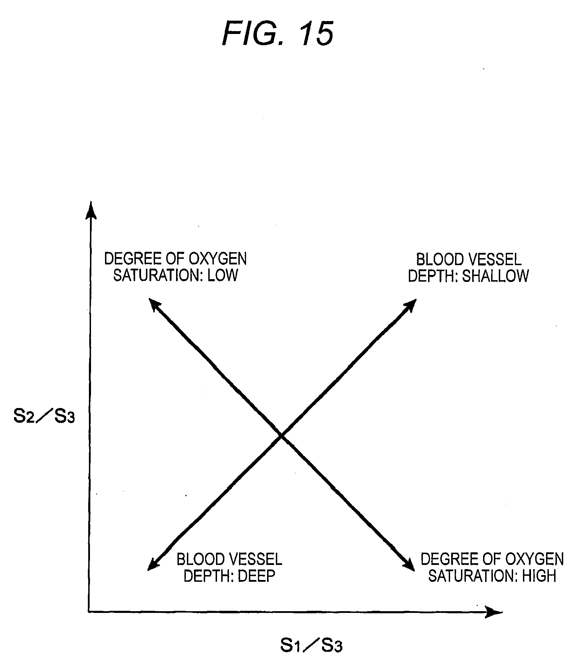

Using these characteristics, the oxygen saturation of the observation area and blood vessel depth captured in the observation area can be found in the following manner.
(1) When the laser light having the center wavelength of 445 nm of which the reduced hemoglobin Hb has higher absorbance is radiated, a captured image luminance value S 1 is obtained by detecting a return light component of this laser light.
(2) When the laser light having the center wavelength of 473 nm of which the oxygenated hemoglobin HbO₂ has higher absorbance is radiated, a captured image luminance value S2 is obtained by detecting a return light component of this laser light.
(3) When the laser lights having the center wavelength of 405 nm of which the reduced hemoglobin Hb and the oxygenated hemoglobin HbO₂ have substantially equal absorbance is radiated, a captured image luminance value S3 is obtained by detecting a return light component of this laser light.
(4) The values of S 1 and S2 are standardized by the value of S3. That is, the values of S 1/S3 and S2/S3 are obtained.
(5) As shown in Fig. 15, a two-dimensional map in which magnitudes of the value of S1/S3 and the value of S2/S3 are expressed by two orthogonal axes is created. On this two-dimensional map, the obtained values of S1/S3 and S2/S3 are plotted. On the two-dimensional map, the larger the value of S1/S3 is, the higher the oxygen saturation is and the shallower the blood vessel depth is. The smaller the value of S1/S3 is, the lower the oxygen saturation is and the deeper the blood vessel depth is. Also, the larger the value of S2/S3 is, the lower the oxygen saturation is and the shallower the blood vessel depth is. The smaller the value of S2/S3 is, the higher the oxygen saturation is and the deeper the blood vessel depth is. According to these relationships, information of the oxygen saturation (high/low) and blood vessel depth in the observation area can be obtained.

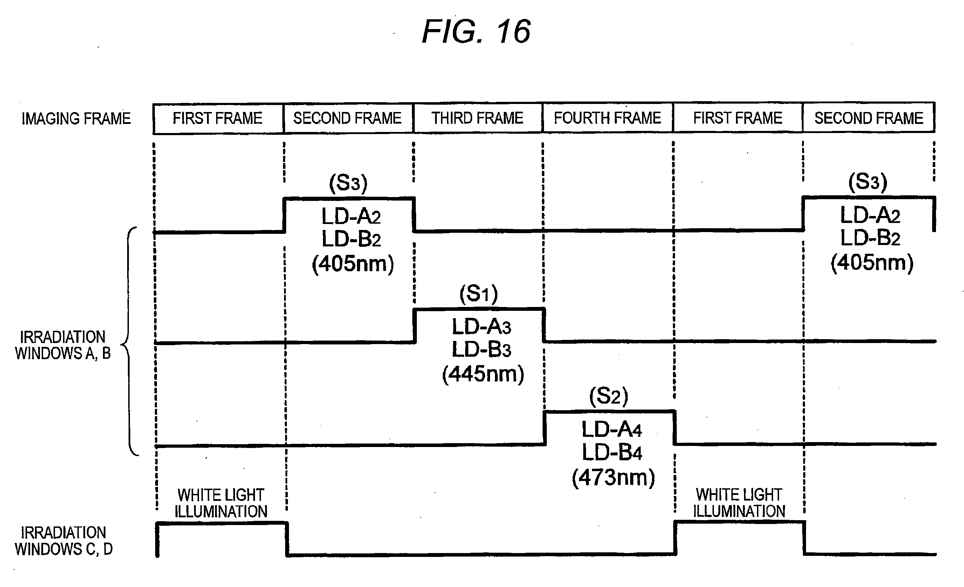

The captured image luminance values S1, S2 and S3 is obtained from captured image data which is obtained by capturing the observation area while switching the lights emitted from the respective irradiation windows. Fig. 16 shows a first control example for switching the lights emitted from the respective irradiation windows in response to the imaging frames. Observation images in the first control example are constituted of first to fourth frames. The first frame is an observation image under white illumination from the irradiation windows C and D, which provides a normal observation image. The second frame is an observation image with the narrow bandwidth lights (center wavelength of 405 nm) of LD-A2 and LD-B2 radiated from the irradiation windows A and B. The third frame is an observation image with the narrow bandwidth lights (center wavelength of 445 nm) of LD-A3 and LD-B3 radiated from the irradiation windows A and B. The fourth frame is an observation image with the narrow bandwidth lights of LD-A4 and LD-B4 (center wavelength of 473 nm) radiated from the irradiation windows A and B.

In the first control example, S3 is obtained from the second frame, S1 is found from the third frame, and S2 is obtained from the fourth frame.

Also, if a difference in receiving sensitivity between the B and G of the imaging device 21 is used, the captured image luminance values S1, S2 and S3 can be obtained by radiating two different kinds of laser lights simultaneously.

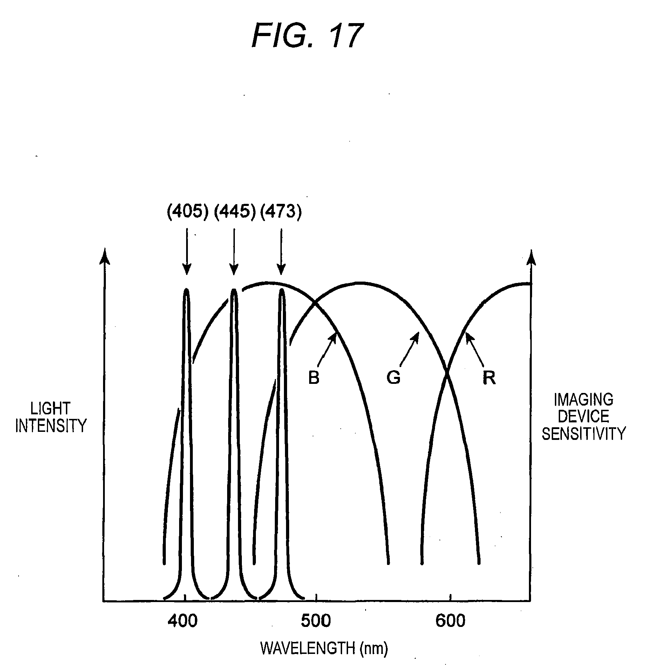

Fig. 17 shows the spectral profiles of the respective laser lights (the center wavelengths of which are respectively 405nm, 445 nm and 473 nm), and the B, G and R detection sensitivities of the imaging device 21. As shown in Fig. 17, the B light detection range of the imaging device 21 contains the wavelength components of the respective laser lights and, the G light detection range contains only the 473 nm wavelength component. Thus, an observation area is captured while the laser light having the center wavelength of 445 nm and the laser light having the center wavelength of 473 nm are simultaneously radiated, and the G light detection signal is subtracted from the then B light detection signal. Thereby, only the laser light component (S1) having the center wavelength of 445 nm can be selectively extracted. Also, the laser light component (S2) having the center wavelength of 473 nm can be obtained from the G light detection signal.

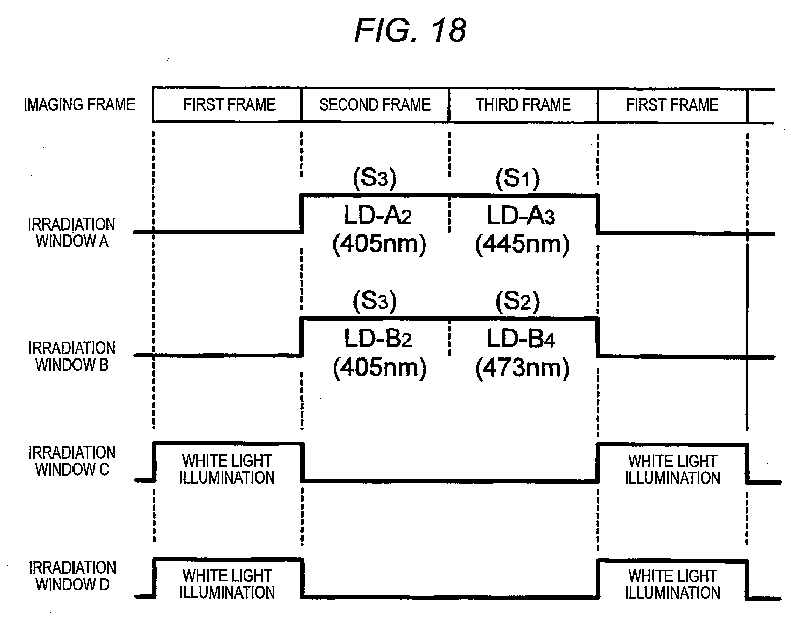

Fig. 18 shows a second control example for switching the lights emitted from the respective irradiation windows in response to the imaging frames.

Observation images in the second control example are constituted of first to third frames. The first frame is an observation image with white lights emitted from the irradiation windows C and D, which provides a normal observation image. The second frame is an observation image with the narrow bandwidth lights (center wavelength of 405 nm) of LD-A2 and LD-B2 radiated from the irradiation windows A and B. The third frame is an observation image which is obtained when the narrow bandwidth light of LD-A3 (center wavelength of 445 nm) from the irradiation window A and the narrow bandwidth light of LD-B4 (center wavelength of 473 nm) from the irradiation window B are simultaneously radiated.

As in the second control example, the narrow bandwidth lights having different wavelengths are radiated simultaneously within the same frame. Therefore, the frame rate can be enhanced. Thereby, the moving image can be displayed more smoothly. Especially, in the observation of the blood oxygen level, since variations in the state of a living body can be detected with high responsibility, failure to detect a lesion can be eliminated, thereby contributing to enhancement of the diagnosis precision. Also, when the endoscope leading end portion is moved, the image capturing screen also moves, which would disturb the display on the screen. However, since the frame rate is enhanced, the disturbance of the display can be reduced, which makes it easy to confirm an observation position within the subject.

Also, when S1 and S3 are obtained, LD1 and LD2 may be used. In this case, there is a possibility that fluorescence is generated from the fluorescent member 57. However, this fluorescence appears on the long wavelength side that is distant from the wavelength band of the laser lights of LD1 and LD2. Therefore, by using only the B imaging signals of the R, G and B color imaging elements as detection values, the fluorescence component can be selectively removed. Therefore, only the return light components of the laser lights of LD1 and LD2 can be detected.

### <Sixth Irradiation Pattern>

The sixth irradiation pattern is an irradiation pattern for carrying out an infrared light observation. The light source control section 49 causes one or both of the laser light sources LD1 and LD2 to emit the laser light (center wavelength of 405 nm) and/or the laser light (center wavelength of 445 nm), and causes the white light or the white light and the laser light (narrow bandwidth light) of LD1 to be radiated from the irradiation windows C and D. Also, the light source control section 49 causes LD-A5 and LD-B5 to emit the laser lights (center wavelength of 780 nm), and causes the laser lights of LD-A5 and LD-B5 to be radiated from the irradiation windows A and B.

In this case as well, the lights radiated from the irradiation windows C and D are used as white light illumination, while the lights radiated from the irradiation windows A and B are used as illumination lights for infrared light observation. Also, as shown in Fig. 13, the timings of radiation of lights from the irradiation windows A, B and the irradiation windows C, D are set alternately every imaging frame.

The laser light having the center wavelength of 780 nm is used to observe blood information of the mucous tissue deep layer and is able to carry out infrared light observation and blood vessel navigation using ICG. ICG is connected to protein in blood and, as shown in Fig. 11, absorbs near infrared light having a wavelength of 750 nm to 850 nm to generate a near infrared fluorescence which is shown by ICG fluorescent profile PF1.

With this irradiation pattern, in addition to the white light from the irradiation windows C and D, the near infrared lights can be radiated from the irradiation windows A and B. Therefore, a normal observation can be made and, especially, there can be extracted blood information of the mucous membrane tissue deep layer which is hard to obtain under illumination of visible light.

For example, in the case where this light projection unit is applied to an endoscope navigation system for obtaining position information of a blood vessel in the periphery of bronchial tubes, the laser light having the center wavelength of 780 nm is radiated onto ICG injected into the blood vessel. As a result, fluorescence having a peak wavelength of 830 nm and having a broad spectral characteristic is generated in a portion where blood and ICG react with each other. Therefore, by using the generated fluorescence as a mark, the position accuracy can be enhanced, and thus an accurate treatment can be carried out. Further, since plural light projection units are used, by combining the lights from the light projection units, light radiation of high intensity is possible.

### <Seventh Irradiation Pattern>

The seventh irradiation pattern is an irradiation pattern for carrying out a drug fluorescence observation using a fluorescent drug with a near ultraviolet light as an excitation light. The light source control section 49 causes one or both of the laser light sources LD1 and LD2 to emit the laser light (center wavelength of 405 nm) and/or the laser light (center wavelength of 445 nm), and also causes the white light or the white light and the laser light (narrow bandwidth light) of LD1 to be radiated from the irradiation windows C and D. Also, the light source control section 49 causes LD-A1 and LD-B1 to emit the laser lights (center wavelength of 375 nm), and then causes the laser lights of LD-A1 and LD-B1 to be radiated from the irradiation windows A and B. In this case as well, the timings of radiation of beams from the irradiation windows A, B and C, D are set alternately every imaging frame as shown in Fig. 13.

As shown in Fig. 11, a luciferase is excited by the near ultraviolet light having the wavelength of 375 nm to generate fluorescence (having a wavelength of up to 490 nm) which is shown by a luciferase fluorescence profile PF2. With this irradiation pattern, since the near ultraviolet light is radiated in addition to the white light, especially, information of the mucous tissue surface layer, which is hard to obtain by ICG, can be obtained using a blue/green spectrum light of high visibility.

### <Eighth Irradiation Pattern>

The eighth irradiation pattern is an irradiation pattern which combines the fluorescence observation using ICG according to the sixth irradiation pattern and the fluorescence observation using luciferase according to the seventh irradiation pattern to thereby obtain a blood vessel image.

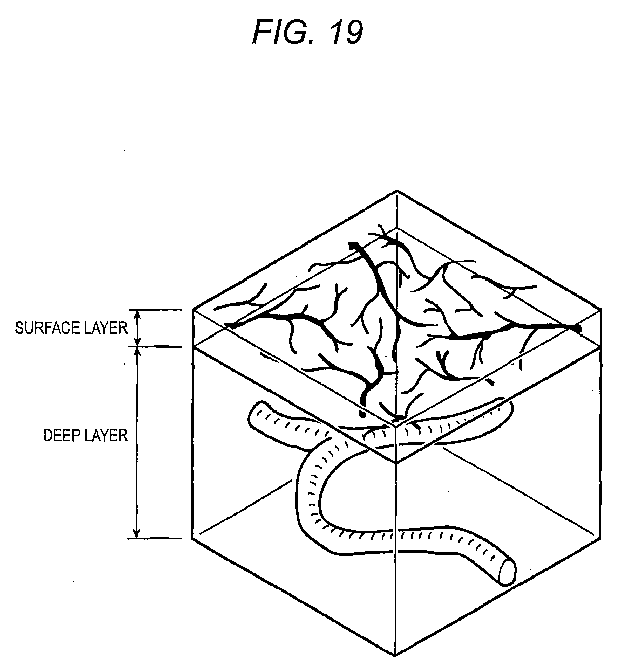

Since the near infrared laser light (the laser lights of LD-A5 and LD-B5) used as the excitation light for ICG reaches from the body surface to the relatively deep layer, the images of blood vessels (as shown in Fig. 19) in a deep layer existing approximately 1 mm to 3 mm deep from the body surface can be obtained from a fluorescence observation image which is captured using ICG, while an image of surface layer blood vessels existing in a surface layer extending approximately 1 mm from the body surface is lowered in sharpness. On the other hand, since the near ultraviolet laser light (the laser lights of LD-A1 and LD-B1) serving as the excitation light of luciferase has short wavelengths, in the luciferase fluorescence image, an image of the surface layer blood vessels existing in the surface layer extending approximately 1 mm from the body surface appears sharply, whereas an image of a deep layer blood vessel cannot be observed.

Then, by using the ICG fluorescence image and luciferase fluorescence image and changing a light amount ratio of the near infrared light and near ultraviolet light, the images of blood vessels existing from the deep layer to the surface layer can be observed properly according to the respective depth ranges.

Also, when only the deep layer blood vessel image is obtained, if only the ICG fluorescence image is obtained, the ICG fluorescence image includes not only information of the deep layer blood vessel image but also information of the surface layer blood vessel image. Therefore, the surface layer blood vessel image appears as unnecessary information. On the other hand, as described above, the luciferase fluorescence image includes only the information of the surface layer blood vessel image.

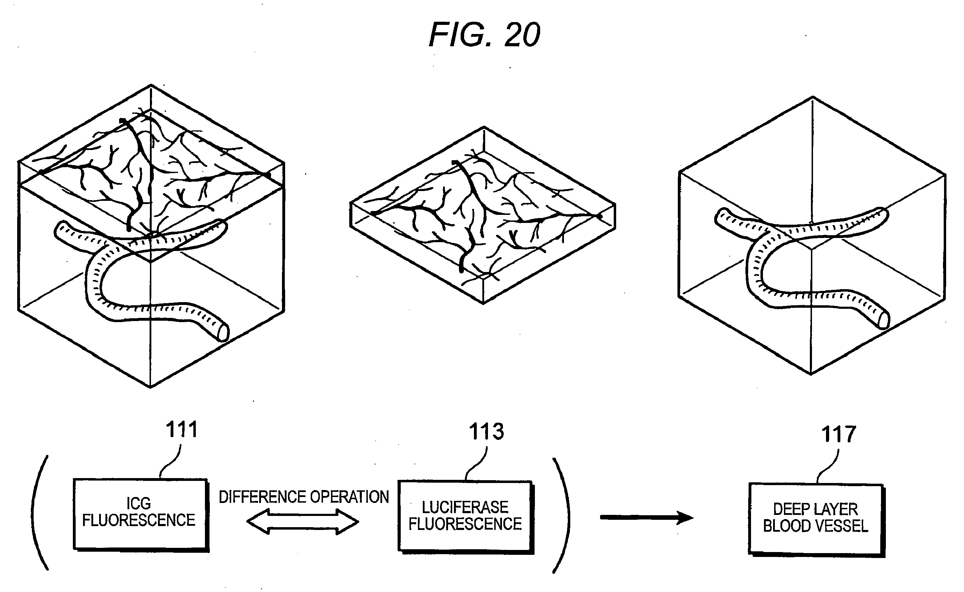

Therefore, when only the deep layer blood vessel image is obtained, as shown in Fig. 20, the deep layer blood vessel image is obtained by subtracting the luciferase fluorescence image from the ICG fluorescence image (subtraction operation). In order to carry out this subtraction properly, the light amount ratio of the near infrared light and near ultraviolet light may be changed so that the magnification of the ICG fluorescence image signal and the magnification of the luciferase fluorescence image signal are equal to each other.

Next, description will be given below on a procedure for operationally processing the ICG fluorescence image and the luciferase fluorescence image to obtain the deep layer blood vessel image.

The ICG fluorescence image radiated according to the sixth irradiation pattern and the luciferase fluorescence image radiated according to the seventh irradiation pattern are temporarily stored in the image processing section 63 shown in Fig. 1. The image processing section 63 performs a blood vessel extraction process for these images.

This blood vessel extraction process is carried out by executing a segment extraction process. As an example, the segment extraction process is carried out by detecting edges and removing an isolated point from the edges detected by the edge detection. Examples of the edge detecting method include a canny method using first derivation.

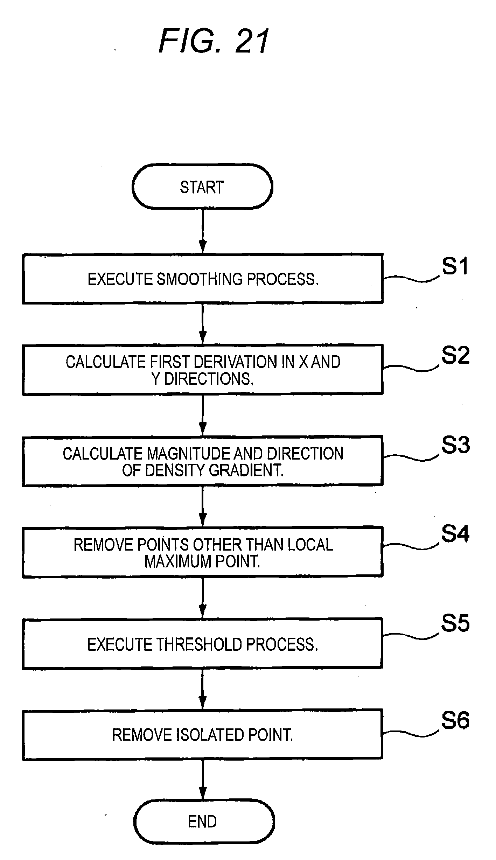

Fig. 21 is a flow chart of the segment extraction process using the edge detection according to the canny method. Firstly, a filter process using a DOG (Derivative of Gaussian) filter is carried out for the ICG fluorescence image signal and the luciferase fluorescence image signal (Sl, S2). This filter process using the DOG filter is constituted of a combination of a Gaussian filter process (smoothing process) for reducing noises and a first-derivative filter process in x and y directions (x and y respectively express the major axis and minor axis of a two-dimensional image) for detecting a density gradient.

For the ICG fluorescence image signal after the filter process and the luciferase fluorescence image signal after the filter process, magnitudes and directions of their density gradients (S3). Then, a local maximum point(s) of the density gradient are extracted, and non-local-maximum points other than the local maximum point(s) are removed (S4).

The extracted local maximum point(s) are compared with a given threshold value, and a local maximum point(s) equal to or larger than the given threshold value are detected as an edge(s) (S5). Further, an isolated point(s), which are the local maximum point(s) and are equal to or larger than the given threshold value but does not constitute a continuous edge(s), are removed (S6). The isolated point removing process is a process for removing an isolated point(s) not proper as a blood vessel from the edge detection result. Specifically, this process detects the isolated point(s) by checking a length(s) of the respective detected edge(s).

The edge detection algorithm is not limited to the above-mentioned algorithm. The edge detection may also be carried out using the LOG (Laplace of Gaussian) filter in which the Gaussian filter process for reducing noises is combined with a Laplacian filter for carrying out a quadratic derivative process to thereby extract an edge(s).

The blood vessel extraction is carried out by executing the segment extraction process using the edge detection. However, this is not limitative but any process, for example, a process using hue or luminance may be used so long as it is a process capable of extracting a blood vessel portion.

By carrying out the blood vessel extraction process in the above manner, with respect to the ICG fluorescence image signal and the luciferase fluorescence image signal, a blood vessel image based on the ICG fluorescence and a blood vessel image based on the luciferase fluorescence. As shown in Fig. 20, this luciferase fluorescence observation image (blood vessel image) 113 represents an image of a surface layer blood vessel existing in a surface layer existing 1 mm from the body surface which is a portion to be observed, while the ICG fluorescence observation image (blood vessel image) 111 includes both the surface layer blood vessel image and an image of a deep layer blood vessel existing in a deep layer existing 1 mm to 3 mm from the body surface.

Then, the image processing section 63 (see Fig. 1) matches the levels of the ICG fluorescence blood vessel image and the luciferase fluorescence blood vessel image, which are generated in the above manner. Then, the image processing section 63 obtains a difference between these two signals. The image signal obtained at this time is a signal of a deep layer blood vessel image 115 (see Fig. 20).

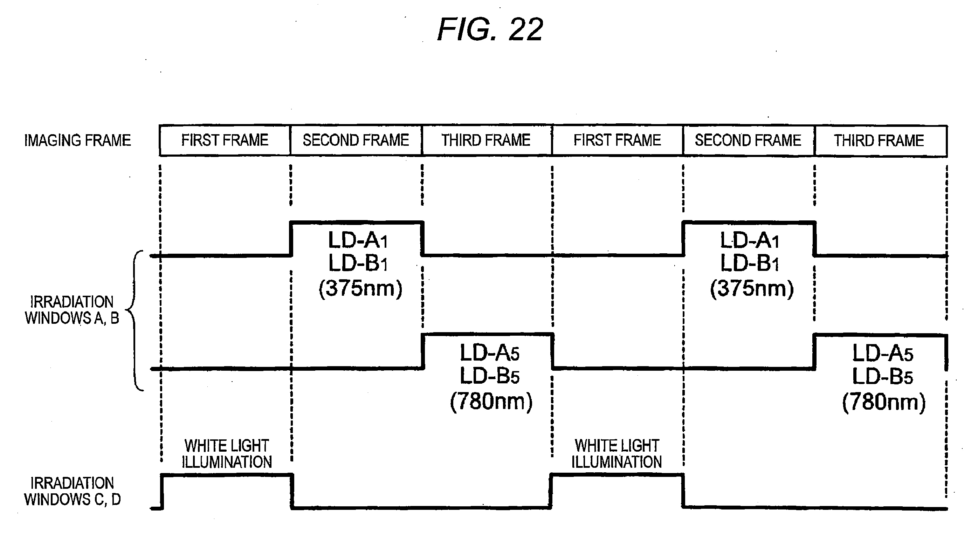

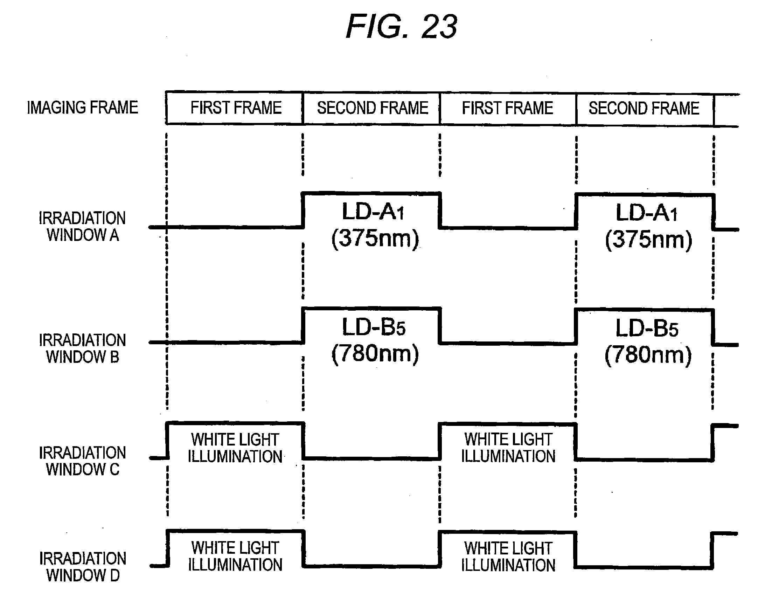

The blood vessel extraction process is carried out based on images which are obtained when narrow bandwidth lights respectively of 375 nm and 780 nm are radiated from the irradiation windows A and B. Fig. 22 shows a first control example for controlling the irradiation pattern. Also, Fig. 23 shows a second control example for controlling the irradiation pattern.

As shown in Fig. 22, according to the first control example, a captured image is constituted of first, second and third imaging frames. The first frame is an observation image under the white light illumination from the irradiation windows C and D, which provides a normal observation image. The second frame is a fluorescence observation image obtained with the narrow bandwidth lights (center wavelength of 375 nm) of LD-A1 and LD-B1 radiated from the irradiation windows A and B, which provides a luciferase fluorescence observation image. The third frame is a fluorescence observation image obtained with the narrow bandwidth lights (center wavelength of 780 nm) of LD-A5 and LD-B5 radiated from the irradiation windows A and B, which provides an ICG fluorescence observation image.

The captured images of the first, second and third frames are temporarily stored in the image processing section 63 (see Fig. 1), where an operation process is carried out for the respective frame images. That is, the image processing section 63 obtains a difference between the luciferase fluorescence observation image 113 of the second frame and the ICG fluorescence observation image 111 of the third frame shown in Fig. 20, thereby obtaining the deep layer blood vessel image 117 shown in Fig. 20. The thus-obtained deep layer blood vessel image 117 is displayed on the display section 15 (see Fig. 1) together with the observation image of the first frame which is obtained under the white light illumination.

With the first control example, the deep layer blood vessel image is obtained from the second and third frames. The thus-obtained deep layer blood vessel image is displayed together with the observation image of the first frame which is obtained under the white light illumination. Thereby, the structure of the tissue deep layer can be observed more clearly.

According to the second control example, as shown in Fig. 23, a captured image is constituted of first and second imaging frames. The first frame is an observation image obtained under the white lights illumination from the irradiation windows C and D, which provides a normal observation image. The second frame provides a luciferase fluorescence observation image and an ICG fluorescence observation image which are obtained when the narrow bandwidth light (center wavelength of 375 nm) of the LD-A1 from the irradiation window A and the narrow bandwidth light (center wavelength of 780 nm) of the LD-B5 from the irradiation window B are radiated simultaneously.

As shown in Fig. 11, while the luciferase excitation light has the wavelength of 375 nm, the luciferase fluorescence profile PF2 in the vicinity of the wavelength of 490 nm is detected as the B light signal of the imaging device, and a portion thereof is detected as a G light signal. On the other hand, while the ICG excitation light has the wavelength of 780 nm, the ICG fluorescence profile PF1 in the vicinity of a wavelength of 820 nm is detected by the imaging device 21 having the above-described configuration structure detects as G, B and R light signals. That is, since the imaging device 21 having the above-described configuration does not have an ordinary IR cut filter for cutting a light component having a wavelength of about 700 nm or more, the ICG fluorescence profile detection signal can be obtained from the respective light receiving pixels G, B and R.

Therefore, according to the imaging device having the above-described configuration, although only the light intensity of the luciferase fluorescence profile PF2 cannot be detected directly, by using the R light signal of the imaging device capable of detecting only the ICG fluorescence profile PF1, it is possible to detect the light intensity of the luciferase fluorescence profile PF2. That is, in order to detect the light intensity of the luciferase fluorescence profile PF2, the R light signal (PF1) of the imaging device is subtracted from the B light signal (PF1 + PF2) of the imaging device to thereby selectively detect only the light intensity of the luciferase fluorescence profile PF2.

With the second control example, since the imaging frame is constituted of the first, second and third frames, the frame rate can be enhanced. Thereby, the display of the moving images can be smoothed further. Especially, in the display of the endoscope observation image, when the endoscope leading end portion is moved, the image capturing screen moves and thus the display of the image would be disturbed. However, since the frame rate is enhanced, the disturbance of the display can be reduced. Thereby, an observation position within a subject can be made easy to confirm.

Next, description will be given below on modifications of the above-described endoscope apparatus.

### <First Modification>

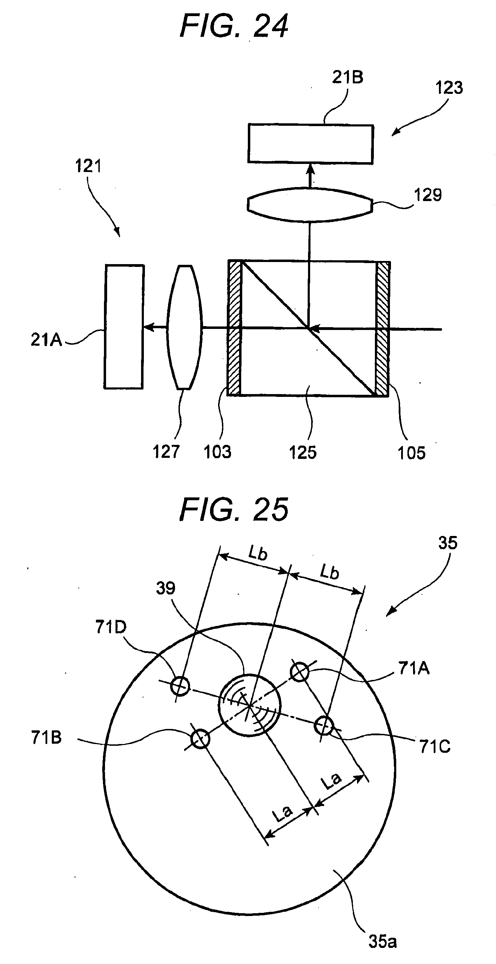

In this modification, an imaging optical system has a structure shown in Fig. 24. Description will be given on the structure of this modification with reference to the case where this structure is used in the above-mentioned eighth irradiation pattern. As shown in Fig. 24, the imaging optical system includes a first imaging system 121 which captures an image of an ICG fluorescence emitted from an area to be observed with the radiation of the near infrared excitation light to generate the image signal of the ICG fluorescence in the area to be observed, and a second imaging system 123 which captures an image of a luciferase fluorescence emitted from the area to be observed with the radiation of a near ultraviolet excitation light to generate the image signal of the luciferase fluorescence of the area to be observed and also captures a normal observation image reflected from the area to be observed under illumination of white light to generate the normal image signal of the area to be observed.

The first imaging system 121 includes a dichroic prism 125, a near infrared light cut filter 103, a first image forming optical system 127, and a first imaging device 21A. The dichroic prism 125 allows the ICG fluorescence image from the area to be observed to pass therethrough. The near infrared light cut filter 103 allows the ICG fluorescence image having passed through the dichroic prism 125 to pass therethrough and cuts a near infrared excitation light having passed through the dichroic prism 125. The first image forming optical system 127 forms the ICG fluorescence image having passed through the near infrared light cut filter 103. The first imaging device 21A captures the ICG fluorescence image formed by the first image forming optical system 127.

The second imaging system 123 includes the dichroic prism 125, a second image forming optical system 129, and a second imaging device 21B. The dichroic prism 125 reflects a normal observation image, which is reflected from the area to be observed, and a luciferase fluorescence image in the right angle direction. The second image forming optical system 129 forms the normal observation image and the luciferase fluorescence image, which are reflected by the dichroic prism 125. The second imaging device 21B captures the normal observation image and the luciferase fluorescence image, which are formed by the second image forming optical system 129 at different timings.

Also, an ultraviolet light cut filter 105 for cutting incidence of a near ultraviolet light is provided on a light incident surface of the dichroic prism 125,. The ultraviolet light cut filter 105 cuts the wavelength bandwidth of 375 nm of the ultraviolet light.

The second imaging device 21B includes an IR cut filter for cutting the wavelength components of about 700 nm or more on a light receiving surface thereof. Thus, the second imaging device 21B does not detect the image of ICG fluorescence which is in a wavelength range in the vicinity of 820 nm. Therefore, even if a narrow bandwidth light having a center wavelength of 375 nm and a narrow bandwidth light having a center wavelength of 780 nm are radiated simultaneously, the second imaging device 21B can detect the luciferase fluorescence image free from the influence of the ICG fluorescence image.

In the imaging optical system according to this modification, by detecting return lights from the area to be observed using the two imaging devices, especially, a weak light can be detected with high accuracy by the highly-sensitive first imaging device 21A separately from the second imaging device 21B which detects a relatively high-intensity light. That is, since the ICG fluorescence image is weak, in the case where the first imaging device 21A for detecting the ICG fluorescence image is configured of a highly-sensitive imaging device, it is possible to obtain an observation image of higher quality.

The specific structure of the first imaging device 21A will be described below. All of light receiving pixels, which are arranged in the Bayer format and detect B, G and R, are used to detect the ICG fluorescence image, and 2 x 2 pixels are mixed (binning). The pixel mixture may be performed by reading and adding signal charges accumulated in photo diodes of the light receiving pixels. Or, the pixel mixture may be performed in such a manner that after luminance values of the respective pixels are processed into data, the data are added by operation.

Also, the first imaging device 21A may be configured to be larger in pixel size than the second imaging device 21B, that is, the first imaging device 21A may be configured such that the light receiving areas of the individual photo diodes thereof are large. Also, the first imaging device 21A may also be configured of a monochrome imaging device which does not have color filters.

As described above, even if the light for exciting luciferase and the light for exciting ICG are radiated simultaneously, the respective fluorescence images can be detected separately by the first and second imaging devices 21A and 21B without carrying out the operation between the detection colors. This makes it possible to facilitate the simultaneous detection of different kinds of fluorescence images. This configuration is not limitedly applied to the eight irradiation pattern, but may also be applied to any of the first to seventh irradiation patterns.

### <Second Modification>

In this modification, an irradiation window for emitting illumination light having a short wavelength is disposed nearer to an observation window than other irradiation windows. This configuration can reduce light amount unevenness, that is, prevents such a phenomenon that light amounts of respective observation wavelengths are different at a time of near distance capturing.

Fig. 25 shows the leading end surface of the endoscope leading end portion. As shown in Fig. 25, two pairs of radiations windows are disposed on the leading end surface 35a of the endoscope leading end portion 35. Specifically, two pairs of irradiation windows are disposed so that an object lens unit 39 is located at a center of the two pairs of irradiation windows and that the irradiation windows of each pair are opposed to each other across the object lens unit 39. The two pairs of irradiation windows include a pair of light projection units 71A, 71B for emitting short wavelength light and a pair of light projection units 71C, 71D for emitting white light.

The light projection units 71A and 71B are respectively located at positions distant by a distance La from the observation window center of the object lens unit 39. The light projection units 71C and 71D are respectively located at positions distant by a distance Lb being longer than the distance La from the observation window center. That is, the light projection units 71A and 71B are disposed so as to be located inside the light projection units 71C and 71D.

Now, description will be given below on an advantage which is provided by the above described configuration, that is, such a configuration that when a pair of first irradiation windows and a pair of second irradiation windows for emitting light having a shorter wavelength than the pair of first irradiation window are disposed on the leading end surface 35a of the endoscope leading end portion 35, the pair of second irradiation windows are disposed inside the pair of first irradiation windows.

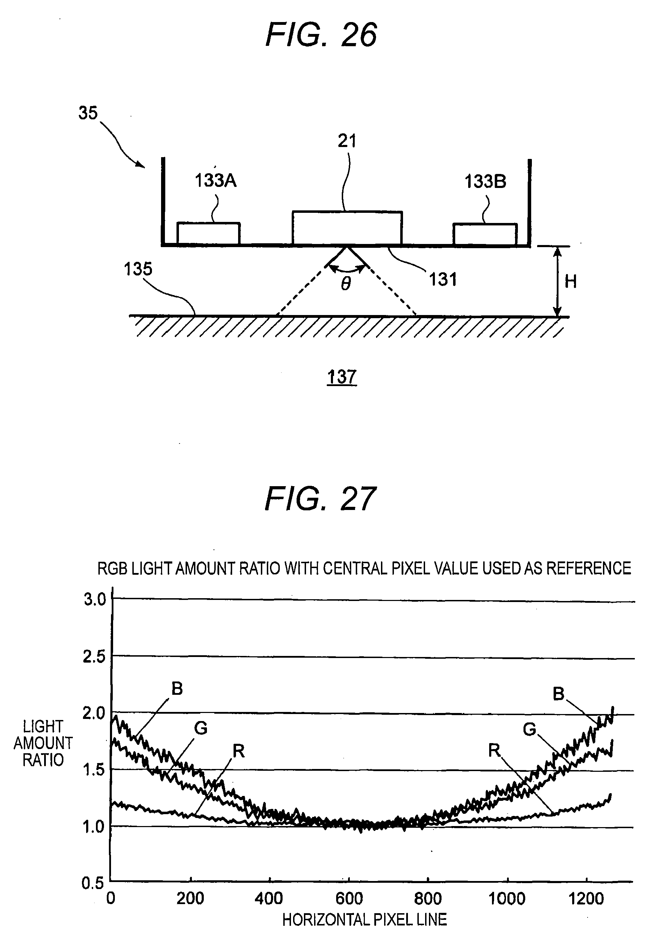

In enlarged observation of the endoscope, a subject is captured while a distance between the endoscope leading end and subject is about 1 mm to 3 mm. In this case, while a normal angle of view of the endoscope is about 120° to about 140°, the angle of view in the enlarged observation is mostly about 50° to about 60°. Fig. 26 shows a schematic section view of the endoscope leading end portion 35. In the case where close capturing with a distance H is carried out from an observation window 131 having the imaging device 21 shown in Fig. 26, light reaching a central portion of a screen is less in amount than light reaching a peripheral portion of the screen, which causes unevenness in light amount of the observation image.

The light amount unevenness occurs differently for the respective colors because of the following reasons. That is, of lights output from irradiation windows 133A and 133B which are opposed to each other across the observation window 131 and which are equidistant from the observation window 131, a red light component having a relatively long wavelength is not only reflected by a surface of an area to be observed 135 which is a living tissue, but also scattered by a living body inside portion 137. As a result, the scattered light reaches the central portion of the observation screen. On the other hand, a blue light component having a short wavelength attenuates soon in the living body inside portion 137 and only light reflected by the living body surface 135 reaches the central portion of the observation screen.

Therefore, Fig. 27 shows a graph showing lights of the respective colors detected through the observation window 131. Fig. 27 shows the light amount ratio distributions of the R, G and B detected light with the central pixel values of the observation screen as a reference. The red light R becomes flat in the vicinity of a light amount ratio of 1.0 at any position in the horizontal pixel line of the observation screen. The red light R provides small unevenness in light amount. On the other hand, as compared with the center of the horizontal pixel line, the green light component G and blue light component B respectively having shorter wavelengths than the red light R have the large light amount ratio in the peripheral portions, that is, portions near the irradiation windows 133A and 133B. In other words, as the wavelength of the light component becomes shorter, the light amount ratio of the peripheral portion becomes larger and the light amount unevenness increases.

Therefore, the irradiation window for emitting light having a shorter wavelength is disposed nearer to the observation window, and thereby, adverse affect of the light amount unevenness can be avoided.

### <Third Modification>

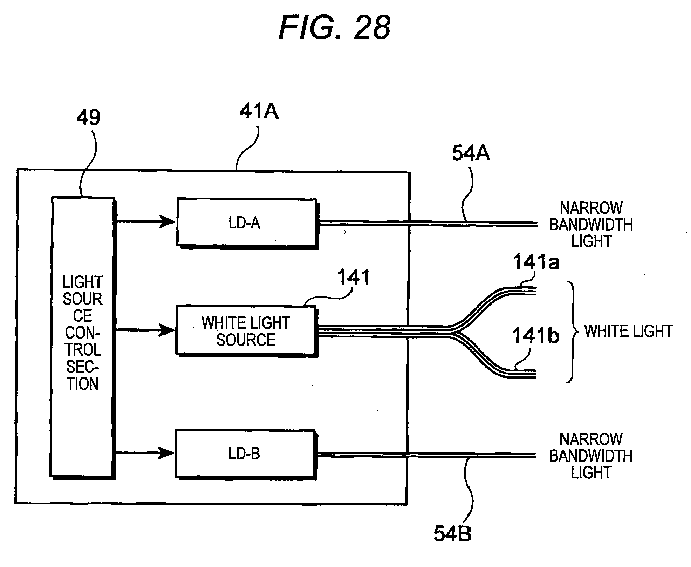

In this modification, as shown in Fig. 28, a white light source 141 such as a xenon light source, a halogen light source, or a white light emission diode light source is used in the light source device 41A as a light source for generating the white light. The emission light from the white light source 141 is guided to the endoscope leading end portion by a fiber bundle 143 which is a bundle constituted of a large number of optical fibers. The white light source 141 is diverged in the intermediate portion into two systems. Specifically, instead of the light projection units 71C and 71D shown in Fig. 1, two bundle end portions 141a and 141b are provided.

The white light of the white light source 141 is emitted from these bundle end portions 141a and 141b under the control of the light source control section 49. With this configuration, white light having a broad spectrum can be radiated onto an area to be observed, to thereby obtain an observation image having high color rendering properties.

### <Fourth Modification>

In this modification, a white light source and a rotating optical filter are used in of the light source device 41B as a light source for radiating the narrow bandwidth light.

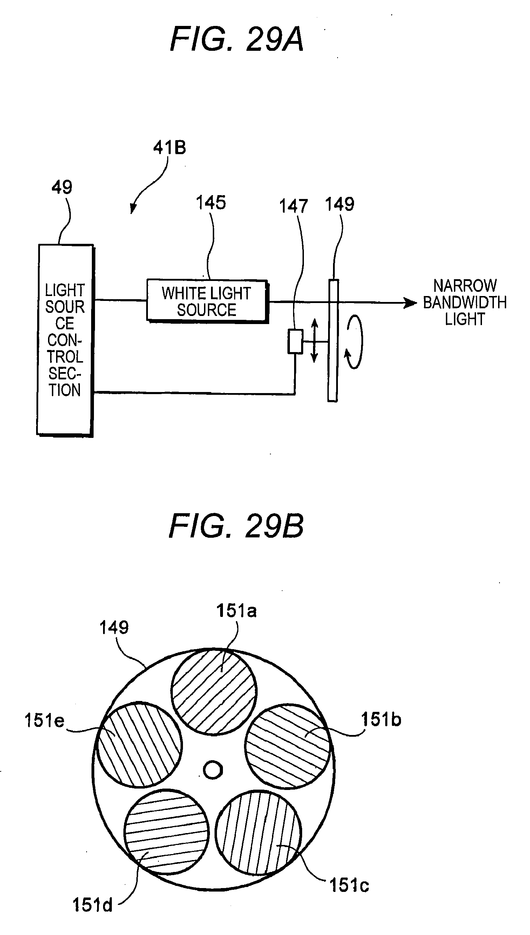

Fig. 29A is a partially schematic configuration view of the light source device 41B. Fig. 29B is a plan view of the rotating optical filter.

This light source device 41B includes a white light source 145, a rotating optical filter 149 driven and rotated by a motor 147, and a light source control section 49 for controlling the white light source 145 and motor 147.

As shown in Fig. 29B, the rotating optical filter 149 includes narrow bandpass filters 151a, 151b, 151c, 151d and 151e which are used to extract narrow bandwidth lights having different spectra from each other. For example, if the respective narrow bandpass filters 151a, 151b, 151c, 151d and 151e are configured by band pass filters which respectively transmit their associated lights respectively having wavelengths of 375 nm, 405 nm, 445 nm 473 nm and 780 nm, the respective narrow bandpass filters 151a, 151b, 151c, 151d and 151e can supply the narrow bandwidth lights to the endoscope leading end portion instead of the previously described first and second laser light sources LD-A and LD-B.

With this light source device 41B, by only changing the optical properties of the narrow bandpass filters, narrow bandwidth lights having arbitrary wavelength bands can be generated. Thereby, plural kinds of spectral lights can be supplied to the irradiation windows at a low cost.

### <Fifth Modification>

This modification has a structure in which light emitting elements are mounted on the endoscope leading end portion.

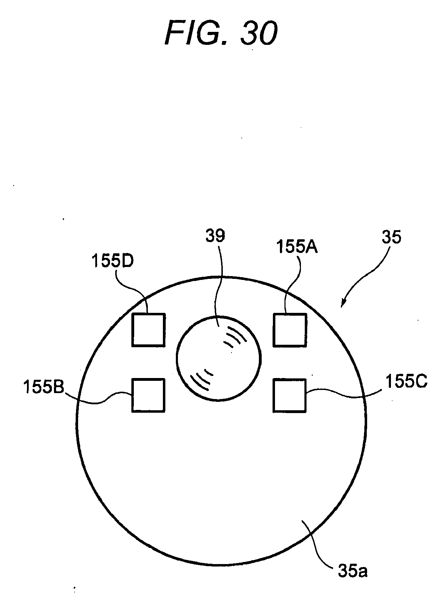

Fig. 30 shows the leading end surface of the endoscope leading end portion. As shown in Fig. 30, a pair of light emitting elements 155A, 155B and a pair of light emitting elements 155C, 155D are so that an observation window disposed on the leading end surface 35a of the endoscope leading end portion 35, that is, an object lens unit 39 is provided at a center of the light emitting elements and that the light emitting elements of each pair are opposed to each other across the objective lens unit 39. The respective light emitting elements are constituted of a semiconductor light emitting element such as a light emitting diode or a small-type semiconductor laser.

With this structure, since the endoscope leading end portion 35 mounts the light emitting elements thereon, the lights emitted from the light source portion need not be guided to the irradiation windows, thereby to prevent loss of light due to beam transmission. This makes it possible to obtain illumination light having high efficiency and high luminance. Also, since wiring within the endoscope insertion portion is simplified, the manufacturing process of the endoscope can be simplified, and the freedom of design thereof can be enhanced, which can contribute to the realization of the further reduced diameter of the endoscope leading end portion.

As described above, since the endoscope apparatus is configured such that, by combining plural light projection units, white light and plural narrow bandwidth lights having different spectra can be radiated individually or simultaneously. Thereby, a normal observation under white light illumination as well as a special light observation such as a narrow bandwidth light observation and a fluorescence observation can be performed in good illumination environments. Also, since the narrow bandwidth light is prevented from passing through the fluorescent member for generating the white light, the light can be radiated with its high intensity being kept as it is without involving any extra wavelength component.

The endoscope apparatus having this configuration is not limited to the above-mentioned embodiments. The invention also presumes that one person skilled in the art modify and apply the above embodiments based on the description of the specification and known technology. Of course, such modification and application will fall within the scope of the protection of the invention.

For example, the light projection units may be set at positions so that a line connecting the light projection units 71A and 71B intersects at right angles with a line connecting the light projection units 71C and 71D. Alternatively, the light projection units may be set at positions so that such the two connecting lines do not intersect with each other. Also, the objective lens unit 39 serving as the observation window may be set at a position which is not included on the respective connecting lines.

Further, light diffusion plates may be disposed on the light emission ends of the light projection units 71A to 71D, and lights may be emitted from the entire light diffusion plate. In this case, even if light is emitted from one of the light projection units, the emission light is diffused by the light diffusion plate. Thus, the light can be radiated in response to the wide area of the light diffusion plate.

Also, plural kinds of wavelength lights may be emitted from the respective irradiation windows of the light projection units 71A and 71B simultaneously.

Here, in the above-mentioned embodiment, the light projection units 71A to 71D are applied to the endoscope apparatus. However, this is not limitative but the light projection units 71A to 71D may be applied to other kinds of medical equipment such as a rigid scope, a scope endoscope, various kinds of operating equipment, or a capsule-type electronic endoscope.

As described above, the specification at least discloses the followings.
(1) According to an aspect of the invention, an endoscope apparatus includes an endoscope insertion portion, a light source section, and a light source control section. The endoscope insertion portion is configured to be inserted into a subject. The light source section supplies light to the endoscope insertion portion. A first irradiation portion that radiates white light onto the subject, a second irradiation portion that radiates to the subject narrow bandwidth light having a narrower wavelength bandwidth than the white light, and an observation window that is used to observe the subject are disposed on a leading end surface of the endoscope insertion portion. Each of the first and second irradiation portions includes a pair of irradiation windows for emitting lights therefrom. A straight line passing through a center point of the observation window and bisecting the leading end surface of the endoscope insertion portion is defined as a boundary line. The pair of irradiation windows of the first irradiation portion are disposed on the leading end surface on both sides of the boundary line. The pair of irradiation windows of the second irradiation portion are disposed on the leading end surface on the both sides of the boundary line. The light source control section is configured to individually change spectra of the lights radiated from the respective irradiation windows of the second irradiation portion.
   With the above configuration, the pair of irradiation windows of the first irradiation portion are disposed on the leading end surface of the insertion portion on the both sides of the boundary line. The pair of irradiation windows of the second irradiation portion are disposed on the leading end surface of the insertion portion on the both sides of the boundary line. The spectra of the lights radiated from the respective irradiation windows of the second irradiation portion can be changed individually. Thereby, the narrow bandwidth lights having the different spectra from each other can be emitted simultaneously from the second irradiation portion. Thus, in addition to the normal observation under the white light illumination and the special light observation using the narrow bandwidth lights, such an observation can be performed that the narrow bandwidth lights having the different spectra are radiated.
(2) The endoscope apparatus of (1) may further include an imaging device that captures an image of the subject through the observation window. The light source control section may cause the narrow bandwidth lights having different spectra from each other to be simultaneously emitted from the respective irradiation windows of the second irradiation portion in synchronous with imaging frames of the imaging device.
   With this configuration, imaging signals output from the imaging device can include an imaging frame in which the narrow bandwidth lights having the different spectra from each other are radiated simultaneously from the second irradiation portion.
(3) In the endoscope apparatus of (1), the light source control section may cause the white light to be emitted from the first irradiation portion at a first imaging frame of the imaging device. The light source control section may cause the narrow bandwidth lights having the different spectra from each other to be emitted simultaneously from the respective irradiation windows of the second irradiation portion at a second imaging frame of the imaging device.
   With this configuration, the imaging signal including the first imaging frame which is obtained under the white light illumination and the second imaging frame which is obtained under illumination of the plural narrow bandwidth lights can be obtained. By combining these imaging frames, information of a desired observation image corresponding to an observation purpose can be obtained. For example, the normal observation image under the white light illumination and the special light observation image using the narrow bandwidth light can be observed while comparing them with each other. Thereby, image information which contributes to specifying of an observation portion and to diagnosis of a lesion portion can be supplied.
(4) In the endoscope apparatus of any one of (1) to (3), the imaging device may include plural kinds of light receiving pixels having sensitivities to specific basic color components.
   With this configuration, captured image data can be obtained for each basic color component. Therefore, for example, when a second imaging frame includes plural color components, these color components can be color separated in terms of the basic colors and thus they can be extracted individually. Thereby, an operation can be performed using the captured image data of each basic color component, which facilitates analysis of observations and diagnosis of lesions.
(5) In the endoscope apparatus of any one of (1) to (4), the respective irradiation windows of the first irradiation portion may include fluorescent members which are configured to be excited by the light supplied from the light source section to emit fluorescence.
   With this configuration, the supplied illumination light can be wavelength converted by the fluorescent member. Thereby, the white light can be radiated. Furthermore, due to the light diffusion effect of the fluorescent members, a wide and uniform light intensity distribution can be achieved.
(6) In the endoscope apparatus of any one of (1) to (5), the light source section may include a semiconductor light emitting element as a light source.
   With this configuration, the illumination light having high luminance can be obtained at high efficiency, which facilitates control of the lights radiated from the respective irradiation windows.
(7) In the endoscope apparatus of any one of (1) to (4), the light source section may supply emission light of a xenon light source or a halogen light source to the first irradiation portion.
   With this configuration, since the light from the white light source having broad spectral properties is supplied to the first irradiation portion, the color rendering properties of the white illumination light can be enhanced, which enhances a quality of an image in a normal observation.
(8) In the endoscope apparatus of any one of (1) to (4), the light source section may cause emission light of a xenon light source or a halogen light source to transmit through a narrow bandpass filter that allows only a given narrow-bandwidth wavelength component to pass therethrough, so as to generate the narrow bandwidth light. The light source section may supply the generated narrow bandwidth light to the respective irradiation windows of the second irradiation portion.
   With this configuration, transmitting light from the narrow bandpass filter is used as the narrow bandwidth light to be radiated from the second irradiation portion. Therefore, by only changing the optical properties of the narrow bandpass filter, a narrow bandwidth light having an arbitrary wavelength bandwidth can be obtained. Thereby, plural kinds of spectral lights can be supplied at a low cost.
(9) In the endoscope apparatus of any one of (1) to (8), the light source section may be provided in a leading end surface of the endoscope insertion portion.
   With this configuration, since the light source section is mounted on the leading end surface of the endoscope insertion portion, the light emitted from the light source section need not be guided to the irradiation windows. This makes it possible to obtain the illumination light which does not incur any loss due to light transmission but can provide high efficiency and high luminance.
(10) In the endoscope apparatus of any one of (1) to (9), the pair of irradiation windows of the first irradiation portion may be disposed on the both sides of the observation window with being opposed to each other across the observation window. The pair of irradiation windows of the second irradiation portion may be disposed at positions, which are different from those of the irradiation windows of the first irradiation portion, on the both sides of the observation window with being opposed to each other across the observation window.
   With this configuration, since the irradiation windows are disposed around the observation window, illumination unevenness hardly occurs in an observation image observed through the observation window, to thereby obtain a high quality observation image.

## Claims

1. An endoscope apparatus comprising:
an endoscope insertion portion configured to be inserted into a subject;
a light source section that supplies light to the endoscope insertion portion; and
a light source control section, wherein
a first irradiation portion that radiates white light onto the subject, a second irradiation portion that radiates to the subject narrow bandwidth light having a narrower wavelength bandwidth than the white light, and an observation window that is used to observe the subject are disposed on a leading end surface of the endoscope insertion portion,
each of the first and second irradiation portions includes a pair of irradiation windows for emitting lights therefrom,
a straight line passing through a center point of the observation window and bisecting the leading end surface of the endoscope insertion portion is defined as a boundary line,
the pair of irradiation windows of the first irradiation portion are disposed on the leading end surface on both sides of the boundary line,
the pair of irradiation windows of the second irradiation portion are disposed on the leading end surface on the both sides of the boundary line, and
the light source control section is configured to individually change spectra of the lights radiated from the respective irradiation windows of the second irradiation portion.

2. The endoscope apparatus according to claim 1, further comprising:
an imaging device that captures an image of the subject through the observation window, wherein
the light source control section causes the narrow bandwidth lights having different spectra from each other to be simultaneously emitted from the respective irradiation windows of the second irradiation portion in synchronous with imaging frames of the imaging device.

3. The endoscope apparatus according to claim 2, wherein
the light source control section causes the white light to be emitted from the first irradiation portion at a first imaging frame of the imaging device, and
the light source control section causes the narrow bandwidth lights having the different spectra from each other to be emitted simultaneously from the respective irradiation windows of the second irradiation portion at a second imaging frame of the imaging device.

4. The endoscope apparatus according to claim 3, wherein
the imaging device includes plural kinds of light receiving pixels having sensitivities to specific basic color components.

5. The endoscope apparatus according to any one of claims 1 to 4, wherein the respective irradiation windows of the first irradiation portion include fluorescent members which are configured to be excited by the light supplied from the light source section to emit fluorescence.

6. The endoscope apparatus according to any one of claims 1 to 5, wherein
the light source section includes a semiconductor light emitting element as a light source.

7. The endoscope apparatus according to any one of claims 1 to 4, wherein
the light source section supplies emission light of a xenon light source or a halogen light source to the first irradiation portion.

8. The endoscope apparatus according to any one of claims 1 to 4, wherein
the light source section causes emission light of a xenon light source or a halogen light source to transmit through a narrow bandpass filter that allows only a given narrow-bandwidth wavelength component to pass therethrough, so as to generate the narrow bandwidth light, and
the light source section supplies the generated narrow bandwidth light to the respective irradiation windows of the second irradiation portion.

9. The endoscope apparatus according to any one of claims 1 to 8, wherein
the light source section is provided in a leading end surface of the endoscope insertion portion.

10. The endoscope apparatus according to any one of claims 1 to 9, wherein
the pair of irradiation windows of the first irradiation portion are disposed on the both sides of the observation window with being opposed to each other across the observation window, and
the pair of irradiation windows of the second irradiation portion are disposed at positions, which are different from those of the irradiation windows of the first irradiation portion, on the both sides of the observation window with being opposed to each other across the observation window.
